# EUROPEAN PATENT APPLICATION

(11) **EP 3 786 305 A1**
(43) Date of publication of application: **03.03.2021**
(21) Application number: 18916665.5
(22) Date of filing: 07.05.2018
(51) Int. Cl.: C12Q 1/68, C12Q 1/04, C12N 1/20, C12M 1/00, A61K 35/74, A61P 25/24, C12R 1/01

(54) **BIOMARKER FOR DEPRESSION AND USE THEREOF**

(30) Priority: 24.04.2018 CN 201810371437
(71) Applicant: BGI Shenzhen, Shenzhen, Guangdong 518083 (CN)
(72) Inventor: GUO, Ruijin, Shenzhen, Guangdong 518083 (CN); WANG, Qi, Shenzhen, Guangdong 518083 (CN); JIA, Huijue, Shenzhen, Guangdong 518083 (CN); JU, Yanmei, Shenzhen, Guangdong 518083 (CN)
(74) Representative: Schwarz & Partner Patentanwälte OG
(86) International application number: PCT/CN2018/085908
(87) International publication number: WO 2019/205188

(57) **Abstract**

Provided are a biomarker for depression and the use thereof, comprising at least one selected from the following: *Bacteroides thetaiotaomicron* and/or an analogue thereof, *Butyriyibrio crossotus* and/or an analogue thereof, *Alistipes shahii* and/or an analogue thereof, *Clostridium bolteae* and/or an analogue thereof, *Haemophilus parainfluenzae* and/or an analogue thereof, *Veillonella dispar* and/or an analogue thereof, and *Prevotella copri* and/or an analogue thereof. Also provided are a kit for detecting the biomarker, and a device for detecting or predicting whether a subject has depression or related diseases.

## Description

### CROSS-REFERENCE TO RELATED APPLICATIONS

This application claims priority to Chinese Patent Application No. 201810371437.4 filed on April 24, 2018, the entire content of which is incorporated herein by reference.

### FIELD

The present disclosure relates to the field of biomedicine, in particular to a biomarker for depression and use thereof. Specifically, the present disclosure relates to a biomarker for depression or related diseases, a method for determining depression or related diseases or the risk of having depression or related diseases, a kit and use of the biomarker for depression in the manufacture of a kit.

### BACKGROUND

Depression is one of the most common mental diseases and is often manifested as a long-term persistent depression mood which is obviously beyond the necessary limit, causing behaviors like lacking confidence, avoiding the crowd, even generating a sense of guilt, feeling obvious decrease of body energy, slowing the sensing to time or hardly experiencing happiness from interesting activities. Depression can also cause physical dysfunction of patients, such as sleep disorder, sudden or decreased appetite, paining or the like. It is revealed in large-scale national epidemiological studies that the number of patients suffering from depression in China ranks first in the world. The latest report of the World Health Organization (WHO) showed that more than 300 million people in the world were suffering from depression in 2015, accounting for about 4.3% of the global population. Currently, the population suffering from depression in China accounts for 4.2% of the total population. A report issued by the WHO China Representative Office in March 2017 indicated that one in 10 people in the world have experienced at least once severe depression during the whole lifetime. It is estimated that there are more than 322 million depression patients with different ages worldwide, with at least 54 million Chinese patients.

Currently, depression is mainly determined by physicians according to patients' behaviors, without definite physiological or biochemical indicators as a reference. Moreover, the diagnosis rate of depression in prefecture-level hospitals or above in China is less than 20%, resulting in more than 80% of depression patients misdiagnosed. The existing diagnostic standards cannot achieve early warning.

Therefore, the early diagnosis and research of depression are still needed to be improved. There is an urgent need for further research on biomarkers for depression in the field.

### SUMMARY

This application is completed based on present inventors' discovery of the following facts and problems. Intestinal microbiota is the microbial community that exists in human intestine, forming a "second genome" of human body. The human intestinal microbiota and the host form an interrelated system, in which the intestinal microbes not only degrade the nutrients of food digested, host vitamins and other nutrients, but also promote the differentiation and maturation of intestinal epithelial cells, thereby activating the intestinal immune system and regulating host energy storage and metabolism, thus playing an important role in human digestion and absorption, immune response, metabolic activity or the like. Therefore, the present inventors have screened out biomarkers that are highly correlated with depression through analyzing the intestinal microbes of patients suffering from depression and normal subjects as well as gene sequences of these intestinal microbes. These biomarkers are capable of diagnosing depression or related diseases accurately and further monitoring the treatment effect on depression.

Therefore, embodiments of the present disclosure aim at providing a biomarker for evaluating the risk of having depression or early diagnosis of depression, as well as a method for determining the depression or the risk of having depression, thus overcoming the defects of unable early warning, unable diagnosing depression accurately and monitoring the development trend of depression through the existing depression diagnosis methods. The biomarkers and method proposed in the present disclosure are capable of early diagnosing depression and monitoring development trend of depression, as well as being useful in depression pathological classification.

The biomarker for depression is believed to be valuable for early diagnosis based on the following reasons. First, the biomarker of the present disclosure is specific and sensitive to depression. Second, fecal sample analysis can be accurate, safe, affordable and bring good patient compliance. Further, fecal sample is transportable, and subjects would be willing to participate in the given screening procedure owing to the comfortableness and non-invasive manner of the polymerase chain reaction (PCR)-based tests. Third, the biomarker of the present disclosure can be served as a tool for monitoring the treatment effect on depression patients to detect the response to treatment.

In a first aspect, provided in embodiments is a biomarker. In embodiments of the present disclosure, the biomarker includes at least one selected from the group consisting of:
*Bacteroides thetaiotaomicron* and/or an analogue thereof, wherein the genome sequence of the analogue has 85% or above sequence identity with the genome sequence of the *Bacteroides thetaiotaomicron*,
*Butyriyibrio crossotus* and/or an analogue thereof, wherein the genome sequence of the analogue has 85% or above sequence identity with the genome sequence of the *Butyriyibrio crossotus,*
*Alistipes shahii* and/or an analogue thereof, wherein the genome sequence of the analogue has 85% or above sequence identity with the genome sequence of the *Alistipes shahii,*
*Clostridium bolteae* and/or an analogue thereof, wherein the genome sequence of the analogue has 85% or above sequence identity with the genome sequence of the *Clostridium bolteae,*
*Haemophilus parainfluenzae* and/or an analogue thereof, wherein the genome sequence of the analogue has 85% or above sequence identity with the genome sequence of the *Haemophilus parainfluenzae*,
*Veillonella dispar* and/or an analogue thereof, wherein the genome sequence of the analogue has 85% or above sequence identity with the genome sequence of the *Veillonella dispar*, and
*Prevotella copri* and/or an analogue thereof, wherein the genome sequence of the analogue has 85% or above sequence identity with the genome sequence of the *Prevotella copri.*

These biomarkers can all be served as biological markers for depression detection. It is possible to effectively determine whether a subject to be tested suffers from depression or is susceptible to depression (that is, having the risk of depression) or further monitor the treatment effect on depression patients by measuring the existence of one or two or more of these biomarkers in the intestine of the subject. In addition, skilled in the art is able to obtain the normal value or normal range of each biomarker in the intestine through testing and calculating methods in presence of sufficient samples from normal subjects, so as to indicate the amount of individual biomarker in a normal sample. Thereby, measuring the amount of at least one of these biomarkers in a sample of a subject enables to determine whether the subject suffers from depression or is susceptible to depression or enables to further monitor the treatment effect on depression patients . Moreover, it is known to skilled in the art that the genome sequence of an unknown strain or from an unknown nucleic acid source having 85% or above sequence identity with the genome sequence of a known strain refers to that the unknown strain and the known strain belong to the same genus or the genome sequence of the unknown strain can be assigned to the same genus as the known strain, where the unknown strain and the known strain belonging to the same genus have same or similar functions. Thus, these analogs of biomarkers belonging to the same genus as the corresponding biomarkers can be severed as markers for depression as well.

In the present disclosure, the "identity" can be also called "similarity" and refers to the proportion of the same base or amino acid residue between a target sequence (i.e., the sequence to be determined) and a reference sequence (i.e., a known sequence) during the sequence alignment.

In embodiments of the present disclosure, the biomarker is at least one selected from the group consisting of *Bacteroides thetaiotaomicron* VPI-5482, *Butyriyibrio crossotus* DSM 2876, *Alistipes shahii* WAL 8301, *Clostridium bolteae* ATCC BAA-613, *Haemophilus parainfluenzae* ATCC T3T1, *Haemophilus parainfluenzae* ATCC 33392, *Veillonella dispar* ATCC 17748 and *Prevotella copri* DSM 18205. These biomarkers are representative strains of *Bacteroides thetaiotaomicron*, *Butyriyibrio crossotus, Alistipes shahii*, *Clostridium bolteae*, *Haemophilus parainfluenzae*, *Veillonella dispar* and *Prevotella copri,* all of which can be useful in determining depression or related diseases or the risk of developing depression or related diseases.

In embodiments of the present disclosure, the genome sequence of the analogue of *Bacteroides thetaiotaomicron* has 95% or above sequence identity with the genome sequence of the *Bacteroides thetaiotaomicron*,
the genome sequence of the analogue of *Butyriyibrio crossotus* has 95% or above sequence identity with the genome sequence of the *Butyriyibrio crossotus,*
the genome sequence of the analogue of *Alistipes shahii* has 95% or above sequence identity with the genome sequence of the *Alistipes shahii,*
the genome sequence of the analogue of *Clostridium bolteae* has 95% or above sequence identity with the genome sequence of the *Clostridium bolteae*,
the genome sequence of the analogue of *Haemophilus parainfluenzae* has 95% or above sequence identity with the genome sequence of the *Haemophilus parainfluenzae*,
the genome sequence of the analogue of *Veillonella dispar* has 95% or above sequence identity with the genome sequence of the *Veillonella dispar,* or
the genome sequence of the analogue of *Prevotella copri* has 95% or above sequence identity with the genome sequence of the *Prevotella copri.*

It is known to skilled in the art that the genome sequence of an unknown strain or from an unknown nucleic acid source having 95% or above sequence identity with the genome sequence of a known strain indicates that the unknown strain and the known strain belong to the same species or the genome sequence of the unknown strain can be assigned to the same species as the known strain. Thus, skilled in the art can directly obtain genome sequence information from a subject to be tested, followed by aligning the genome sequence from the subject to be tested with the genome sequence from *Bacteroides thetaiotaomicron, Butyriyibrio crossotus, Alistipes shahii*, *Clostridium bolteae, Haemophilus parainfluenzae*, *Veillonella dispar* or *Prevotella copri,* in which 95% or above sequence identity indicates that the subject to be tested suffers from depression or is susceptible to depression.

According to embodiments of the present disclosure, the genome sequence of analogs of individual biomarkers (such as, bacteria identified in the above) having 80% or above coverage and 85% or above identity with the genome sequence of the corresponding biomarkers (i.e., the identified bacteria) indicates that the analogs of bacteria belong to the same genus as the corresponding bacteria, which can be also served as biomarkers for depression. Preferably, the genome sequence of analogs of individual biomarkers (such as, bacteria identified in the above) having 80% or above coverage and 95% or above identity with the genome sequence of the corresponding bacteria indicates that the analogs of bacteria belong to the same species as the corresponding bacteria, which can be also served as biomarkers for depression.

In the present disclosure, the "coverage" also called "overlap", refers to the proportion of sequence length of a target sequence mapped onto the total length of a reference sequence during the sequence alignment.

In a second aspect, provided in embodiments is a method of determining a subject suffering from or having a risk of developing depression or related diseases. According to embodiments, the method includes: (1) obtaining a sample from the subject, (2) measuring relative abundance information of the biomarker as described in the first aspect in the obtained sample, and (3) comparing the measured relative abundance information with a reference data set or a reference value. The method of the present disclosure can be used not only for disease diagnosis in the sense of patent, but also for non-disease diagnosis such as scientific research, personal genetic information enrichment and genetic information database enrichment or the like. Comparing the measured relative abundance information of each biomarker in the subject to be tested with a reference data set or a reference value enables to determine whether the subject suffers from depression or related diseases or has the risk of developing depression or related diseases.

In the present disclosure, the reference data set refers to the relative abundance information of individual biomarkers obtained by using samples from individuals having diagnosed with depression and normal individuals, which is served as a reference of relative abundance of individual biomarkers. In an embodiment of the present disclosure, the reference data set refers to a training set data. According to the present disclosure, the training set and the verification set have meanings well known in the art. In an embodiment of the present disclosure, the training set refers to a dataset including the amounts of individual biomarkers in samples to be tested from a certain number of depression subjects and normal subjects. The validation set refers to an independent dataset used to validate the performance of the training set.

In the present disclosure, the reference value refers to a reference value or a normal value of normal subjects. It is known to skilled in the art that the normal value or normal range (in the absolute value) of each biomarker in samples can be obtained by using testing and calculating methods in presence of sufficient samples from normal subjects. When the level of a biomarker in a sample is detected by an assay method, the detected level of the biomarker in the absolute value in the sample can be directly compared to a reference value, optionally in combination with statistical methods to evaluate the risk of developing depression or related diseases, diagnose or early diagnose depression or related diseases.

In the present disclosure, the depression related diseases refer to diseases that are interrelated with depression, including pre-symptoms or pre-disorders causing depression and subsequent or concurrent symptoms or disorders caused by depression, as well as single episode depression, postpartum depression or the like.

According to embodiments of the present disclosure, the method as described above may further include the following technical features.

In embodiments of the present disclosure, the reference data set includes relative abundance information of the biomarker as described in the first aspect in samples from a plurality of patients suffering from the depression or related diseases and a plurality of normal subjects.

In embodiments of the present disclosure, comparing the measured relative abundance information with a reference data set further includes utilizing a multivariate statistical model to obtain a probability of having the depression or related diseases. The use of multivariate statistical models can achieve rapid and efficient detection.

In embodiments of the present disclosure, the multivariate statistical model is a random forest model.

In embodiments of the present disclosure, the probability of having the depression or related diseases greater than a threshold value indicates that the subject suffers from the depression or related diseases or has the risk of developing the depression or related diseases.

In embodiments of the present disclosure, the threshold value is 0.5.

In embodiments of the present disclosure, decrease of the *Bacteroides thetaiotaomicron* and/or the analogue thereof, the *Alistipes shahii* and/or the analogue thereof or the *Prevotella copri* and/or the analogue thereof compared to the reference value indicates that the subject suffers from the depression or related diseases or has the risk of developing the depression or related diseases, and increase of the *Butyriyibrio crossotus* and/or the analogue thereof, the *Clostridium bolteae* and/or the analogue thereof, the *Haemophilus parainfluenzae* and/or the analogue thereof or the *Veillonella dispar* and/or the analogue thereof compared to the reference value indicates that the subject suffers from the depression or related diseases or has the risk of developing the depression or related diseases.

In embodiments of the present disclosure, measuring relative abundance information of the biomarker is performed by a sequencing technology. Specifically, measuring relative abundance information of the biomarker further includes: extracting nucleic acids from the obtained sample of the subject, constructing a DNA library based on the extracted nucleic acids, followed by sequencing to obtain a sequencing result, and aligning the sequencing result to a reference gene catalogue to determine the relative abundance information of the biomarker. According to an embodiment, aligning the sequencing result to a reference gene catalogue is performed by using at least one of SOAP2 software and MAQ software, thus improving the efficiency of alignment and increasing the efficiency of depression detection. According to the embodiment, a plurality of biomarkers such as at least two biomarkers can be detected at the same time to improve the efficiency of depression detection.

In embodiments of the present disclosure, the reference gene catalogue is obtained by: subjecting samples from a plurality of patients suffering from the depression or related diseases and a plurality of normal subjects to metagenomic sequencing to obtain a non-redundant gene catalogue, and merging the non-redundant gene catalogue with an intestinal microbial gene catalogue to obtain the reference gene catalogue. In the present disclosure, the reference gene catalogue may be a known gene catalogue, such as the published intestinal microbial reference gene catalogue. The reference gene catalogue may be also obtained by metagenomic sequencing samples from a plurality of depression patients and a plurality of normal subjects to obtain a non-redundant gene catalogue, and merging the non-redundant gene catalogue with an intestinal microbial gene catalogue to obtain the reference gene catalogue. Thus, a reference gene catalogue with more comprehensive information is obtained, with more reliable detection results.

In the present disclosure, the non-redundant gene catalogue is explained according to common understanding by skilled in the art, which in short refers to the set of remaining genes after removing redundant genes. Redundant genes usually refer to a plurality of copies of a gene appearing on a chromosome.

In embodiments of the present disclosure, the sample is faeces.

In embodiments of the present disclosure, the sequencing is performed through a second-generation sequencing technology or a third-generation sequencing technology. The sequencing technology is not particularly limited. The second-generation sequencing technology or the third-generation sequencing technology is capable of sequencing in a quick and efficient manner.

In embodiments of the present disclosure, the sequencing is performed by using at least one sequencer selected from Hiseq2000, SOLiD, 454 sequencer and single molecule sequencing sequencers. The sequencer with high-throughput and deep sequencing characteristics used in the present disclosure can be beneficial to the analysis of subsequent sequencing results, especially ensuring the precision and accuracy at statistical testing.

In a third aspect, provided in embodiments is a kit including reagents for detecting the biomarker as described in the first aspect. The kit can be useful in determining the relative abundance of these biomarkers in the intestinal microbiota. Further, whether a subject suffers from or is susceptible to depression can be determined by the relative abundance, and the efficiency of treatment effect for depression patients can be monitored by the relative abundance as well.

In embodiments of the present disclosure, the kit further includes a reference data set or a reference value, wherein the reference data set or the reference value is served as references for relative abundance information of the biomarker. Preferably, the reference data set or the reference value can be attached to a physical carrier, such as an optical disc like a CD-ROM or the like.

In embodiments of the present disclosure, the kit further includes a first computer program product, wherein the first computer program product is configured to obtain the reference data set or the reference value. That is, the first computer program product is configured to obtain a group of reference data sets or reference values for determining a subject suffering from or having a risk of developing depression or related diseases.

In embodiments of the present disclosure, the kit further includes a second computer program product, wherein the second computer program product is configured to perform the method of determining a subject suffering from or having a risk of developing depression or related diseases as described in the second aspect.

In a fourth aspect, provided in embodiments is use of the biomarker as described in the first aspect in the manufacture of a kit for determining a subject suffering from or having a risk of developing depression or related diseases. According to the embodiment, determining a subject suffering from or having a risk of developing depression or related diseases further includes: (1) obtaining a sample from the subject, (2) measuring relative abundance information of the biomarker in the obtained sample, and (3) comparing the measured relative abundance information with a reference data set or a reference value. According to the kit, the relative abundance of these biomarkers in the intestinal microbiota can be determined, thus whether the subject suffers from or is susceptible to depression can be determined by the determined relative abundance and the efficiency of treatment effects on depression patients can be monitored by the determined relative abundance as well.

According to embodiments of the present disclosure, the use of the biomarkers in the manufacture of a kit as described above may further include the following technical features.

In embodiments of the present disclosure, in the use as described above, the reference data set includes relative abundance information of the biomarker as described in the first aspect in samples from a plurality of patients suffering from the depression or related diseases and a plurality of normal subjects.

In embodiments of the present disclosure, in the use as described above, comparing the measured relative abundance information with a reference data set further includes utilizing a multivariate statistical model to obtain a probability of having the depression or related diseases. Preferably, the multivariate statistical model is a random forest model.

In embodiments of the present disclosure, in the use as described above, the probability of having the depression or related diseases greater than a threshold value indicates that the subject suffers from the depression or related diseases or has the risk of developing the depression or related diseases. Preferably, the threshold value is 0.5.

In embodiments of the present disclosure, in the use as described above, decrease of the *Bacteroides thetaiotaomicron* and/or the analogue thereof, the *Alistipes shahii* and/or the analogue thereof or the *Prevotella copri* and/or the analogue thereof compared to the reference value indicates that the subject suffers from the depression or related diseases or has the risk of developing the depression or related diseases, and increase of the *Butyriyibrio crossotus* and/or the analogue thereof, the *Clostridium bolteae* and/or the analogue thereof, the *Haemophilus parainfluenzae* and/or the analogue thereof or the *Veillonella dispar* and/or the analogue thereof compared to the reference value indicates that the subject suffers from the depression or related diseases or has the risk of developing the depression or related diseases.

In embodiments of the present disclosure, in the use as described above, measuring relative abundance information of the biomarker is performed by sequencing. Specifically, measuring relative abundance information of the biomarker further includes: extracting nucleic acids from the obtained sample of the subject, constructing a DNA library based on the extracted nucleic acids, followed by sequencing to obtain a sequencing result, and aligning the sequencing result to a reference gene catalogue to determine the relative abundance information of the biomarker.

In embodiments of the present disclosure, in the use as described above, the reference gene catalogue is obtained by: subjecting samples from a plurality of patients suffering from the depression or related diseases and a plurality of normal subjects to metagenomic sequencing to obtain a non-redundant gene catalogue, and merging the non-redundant gene catalogue with an intestinal microbial gene catalogue to obtain the reference gene catalogue.

In embodiments of the present disclosure, in the use as described above, the sample is faeces.

In embodiments of the present disclosure, in the use as described above, the sequencing is performed through a second-generation sequencing technology or a third-generation sequencing technology.

In embodiments of the present disclosure, in the use as described above, the sequencing is performed by using at least one sequencer selected from Hiseq2000, SOLiD, 454 sequencer and single molecule sequencing sequencers.

In a fifth aspect, provided in embodiments is use of the biomarker as described in the first aspect as a target for screening a medicament for treatment or prevention of depression or related diseases. According to an embodiment of the present disclosure, whether a drug candidate is suitable for treating or preventing depression can be determined according to the influence of the drug candidate on these biomarkers before and after administration.

In a sixth aspect, provided in embodiments is use of the biomarker as described in the first aspect in determining a subject suffering from or having a risk of developing depression or related diseases.

In a seventh aspect, provided in embodiments is a medicament for preventing or treating depression or related diseases. According to embodiments of the present disclosure, the medicament allows relative abundance of *Bacteroides thetaiotaomicron* and/or the analogue thereof, *Alistipes shahii* and/or the analogue thereof or *Prevotella copri* and/or the analogue thereof in a subject to be increased, or allows relative abundance of *Butyriyibrio crossotus* and/or the analogue thereof, *Clostridium bolteae* and/or the analogue thereof, *Haemophilus parainfluenzae* and/or the analogue thereof or *Veillonella dispar* and/or the analogue thereof in a subject to be decreased.

The present disclosure achieved the following beneficial effects. Feces as human excreta, contains not only metabolites of human body, but also intestinal microbes tightly related to the changes in metabolism, immunity and other functions of the human body. It is found that there are significant differences in the composition of intestinal microbes between depression patients and normal subjects according to research of fecal samples, thus the intestinal microbes can be useful in accurately evaluating patients having the risk of developing depression and early diagnosis. The present disclosure proposed a plurality of intestinal bacteria related to depression by comparing and analyzing the intestinal microbes from depression patients and normal subjects, in combination with using metagenomic linkage groups (MLGs) from depression patients and normal subjects with a high quality as a training set. Such intestinal bacteria related to depression can accurately evaluate patients having the risk of developing depression and perform early diagnosis. The method of the present disclosure is more convenient and rapider compared to current diagnostic methods.

The additional aspects and advantages of the present disclosure will be partly given in the following description, of which partly will become apparent from the following description or be understood through the practice of the present disclosure.

### BRIEF DESCRIPTION OF THE DRAWINGS

The above and/or additional aspects and advantages of the present disclosure will become apparent and be easily understood from the description of embodiments in combination with the following drawings, in which:
Figure 1 shows a schematic diagram of an apparatus for determining a subject suffering from or having a risk of developing depression or related diseases according to an embodiment of the present disclosure, in which "a" represents a schematic diagram of the apparatus and "b" represents a schematic diagram of a device for measuring relative abundance information of a biomarker in the apparatus.
Figure 2 shows the difference between counts of two metagenomic linkage groups (MLGs) (p=0.004792, Wilcox test) of patients suffering from depression and normal subjects at a gene level according to an embodiment of the present disclosure.
Figure 3 shows a graph of error rate distribution of 5 times of 10-fold cross-validation in a random forest classifier according to an embodiment of the present disclosure.
Figure 4 is a graph showing a receiver operating curve (ROC) and an area under curve (AUC) of a training set composed of normal subjects and patients suffering from depression based on a random forest model for 8 intestinal biomarkers according to an embodiment of the present disclosure.
Figure 5 is a graph showing a receiver operating curve (ROC) and an area under curve (AUC) of a validation set composed of 30 normal subjects and 10 patients suffering from depression based on a random forest model for 8 intestinal biomarkers according to an embodiment of the present disclosure.

### DETAILED DESCRIPTION

The embodiments of the present disclosure are described in detail below, and examples of embodiments are shown in the drawings. The embodiments described below with reference to the drawings are exemplary, which are intended to explain the present disclosure but should not be construed as limiting the present disclosure.

Aiming at the defects of unable early warning, unable diagnosing depression accurately and monitoring the development trend of depression through the existing depression diagnosis methods, embodiments of the present disclosure provides a biomarker for evaluating the risk of having depression or early diagnosing depression as well as a method for determining depression or the risk of having depression, thus achieving early diagnosis of depression and monitoring development trend of depression and further facilitating depression pathological classification.

### Biomarker

According to a first aspect, the present disclosure in embodiments proposed a biomarker.

The terms used in the present disclosure have the meanings commonly understood by skilled person in the relevant art. However, in order to better understand the present disclosure, some relative terms are defined as follows.

According to the present disclosure, the term "depression" is one of the most common mental diseases and is often manifested as a long-term persistent depression mood which is obviously beyond the necessary limit, causing behaviors like lacking confidence, avoiding the crowd, even generating a sense of guilt, feeling obvious decrease of body energy, slowing the sensing to time or hardly experiencing happiness from interesting activities. Depression can also cause physical dysfunction of patients, such as sleep disorder, sudden or decreased appetite, paining and the like.

According to the present disclosure, the level of biomarker is indicated by relative abundance.

According to the present disclosure, the term "biomarker", also known as "biological marker", refers to a measurable indicator of a biological condition of an individual to be tested. Such a biomarker can be any substance in the individual, as long as the substance is related to the specific biological condition (such as, a disease) in the individual, for example, a nucleic acid marker (also called a gene marker, such as DNA), a protein marker, a cytokine marker, a chemokine marker, a carbohydrate marker, an antigen marker, an antibody marker, a species marker (i.e., a marker for species/genus) and a functional marker (such as, KO/OG marker) or the like. Among them, the meaning of the nucleic acid marker is not limited to existing genes that can be expressed into biologically active proteins, but also includes any nucleic acid fragments, which may be DNA, RNA, modified DNA or RNA, unmodified DNA or RNA, or a combination thereof. In this context, the nucleic acid marker may be referred to as characteristic fragments. In the present disclosure, the biomarker can be interchanged with "intestinal marker", because a plurality of biomarkers closely related to depression proposed in the present disclosure are all existed in the intestine of the individual. Biomarkers are often measured to examine common biological condition, pathogenic process or pharmacological response to therapeutic intervention, which are useful in many scientific fields.

According to embodiments of the present disclosure, high-throughput sequencing may be utilized to analyze fecal samples of normal subjects and depression patients in batches. Sequencing results of the normal subjects and the depression patients are compared to determine specific nucleic acid sequences related to depression. The steps are shortly described as follows.

Sample collection and treatment: nucleic acids from fecal samples collected from normal subjects and depression patients are extracted by using a kit to obtain nucleic acids.

Library construction and sequencing: a DNA library based on the extracted nucleic acids is constructed, followed by high-throughput sequencing to obtain nucleic acid sequences of intestinal microbes in the fecal sample.

The nucleic acid sequences of specific intestinal microbes related to depression are determined through bioinformatics analysis. Specifically, first the nucleic acid sequences (i.e., reads) are aligned with a reference gene catalogue which may be a newly constructed gene catalogue or any database of known sequences, for example, a known non-redundant gene set of human intestinal microbes. Next, the relative abundance of individual genes in nucleic acids from the fecal samples of the normal subjects and the depression patients are determined based on alignment results. Further, the corresponding relationship between the nucleic acid sequences and genes in the reference gene catalogue can be established according to the alignment results, so that the relative abundance of a specific gene in the nucleic acids can be effectively reflected by the number of reads corresponding to the specific gene. Therefore, the relative abundance of all genes in the nucleic acids can be determined through the alignment results and conventional statistical analysis. Finally, the determined relative abundance of individual genes in the nucleic acids from the fecal samples of the normal subjects and the depression patients is statistically analyzed, thus the genes having significantly different relative abundance between the normal subjects and the depression patients are determined. Those significantly differential expressed genes may be regarded as biomarkers of an abnormal condition, that is, a nucleic acid marker.

In addition, the known or newly constructed reference gene catalogue usually contains species information and functional annotations of genes. Thus, based on the determined relative abundance of genes, bacteria in the intestinal microbiota can be further classified according to the species information and functional annotations of genes. Thereby, species relative abundance and function relative abundance of bacteria in the intestinal microbiota can be determined, and the species biomarkers or functional biomarkers indicating an abnormal condition can be further determined. Briefly, determining species biomarkers or functional biomarkers includes aligning the reads of normal subjects and depression patients to a reference gene catalogue, determining species relative abundance and function relative abundance of individual genes in the nucleic acids from the normal subjects and the depression patients respectively based on the alignment results, subjecting the species relative abundance and function relative abundance of individual genes in the nucleic acids from the normal subjects and the depression patients to statistically analysis, and determining species biomarkers and functional biomarkers having significantly different relative abundance between the normal subjects and the depression patients, respectively. According to embodiments of the present disclosure, the relative abundance of genes from the same species and the relative abundance of genes with same functional annotations can be subjected to statistically analysis, for example, summation, averaging, obtaining median value or the like to determine the function relative abundance and the species relative abundance.

Finally, microbial species with significant differences in relative abundance between fecal samples of normal subjects and depression patients is identified, which is served as a biological marker (i.e., biomarker) for depression. The identified microbial species includes *Bacteroides thetaiotaomicron* and/or an analogue thereof, *Butyriyibrio crossotus* and/or an analogue thereof, *Alistipes shahii* and/or an analogue thereof, *Clostridium bolteae* and/or an analogue thereof, *Haemophilus parainfluenzae* and/or an analogue thereof, *Veillonella dispar* and/or an analogue thereof, and/or *Prevotella copri* and/or an analogue thereof. Thus, whether a subject suffers from or has a risk of developing depression can be determined by detecting the existence of at least one of the identified microbial species as described above. Further, treatment effect on depression patients can be monitored by detecting the existence of at least one of the identified microbial species as described above. The term "existence" used herein should be understood in a broad sense, which may refer to qualitative analysis or quantitative analysis of the existence of the corresponding target microbial species in a sample to be tested, and may also refer to subject the quantitative analysis results of the target microbial species in the sample to be tested and a reference (for example, quantitative analysis results of the target microbial species in a sample with a known disease condition obtained by parallel tests) to statistical analysis, or a result obtained through any known mathematical operation. Those skilled in the art can make easy selections according to needs and test conditions. According to embodiments of the present disclosure, measuring relative abundance of the identified microbial species as described above among intestinal microbes enables to determine whether a subject suffers from or has a risk of developing depression and monitor the treatment effect on depression patients.

Detecting the existence of at least one, preferably two or more (i.e., a combination of biomarkers) of the identified microbial species as described above among intestinal microbes in a subject to be tested enables to effectively determine whether the subject suffers from or has a risk of developing depression and further monitor the treatment effect on depression patients. The term "combination of biomarkers" used herein refers to a combination of two or more biomarkers.

Further, with regards to species biomarkers and functional biomarkers, skilled in the art can determine the existence of target species among intestinal microbes by conventional identification methods of bacterial species, for example, identification by 16s rRNA, and skilled in the art can determine the functions of bacterial species by biological activity testing methods.

### Apparatus for determining a subject suffering from or having a risk of developing depression or related diseases

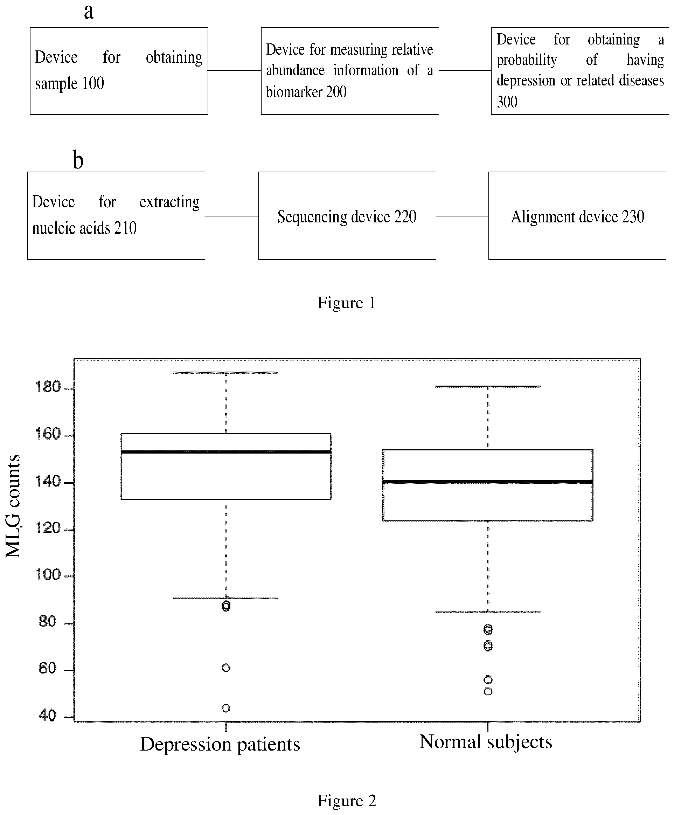

According to another aspect, the present disclosure in embodiments proposes an apparatus for determining a subject suffering from or having a risk of developing depression or related diseases. According to embodiments of the present disclosure, referring to Figure 1, the apparatus includes a device for obtaining sample 100, a device for measuring relative abundance information of a biomarker 200 and a device for obtaining a probability of having the depression or related diseases 300, as shown in panel a of Figure 1. Specifically, the device for obtaining sample is configured to obtain a sample from the subject; the device for measuring relative abundance information of a biomarker is connected to the device for obtaining sample and configured to measure relative abundance information of the biomarker as described in the first aspect; and the device for obtaining a probability of having the depression or related diseases is connected to the device for measuring relative abundance information of a biomarker and configured to compare the measured relative abundance information with a reference data set or a reference value.

According to a specific embodiment, the reference data set includes relative abundance information of the biomarker as described in the first aspect in samples from a plurality of patients suffering from the depression or related diseases and a plurality of normal subjects.

According to a specific embodiment, the device for obtaining a probability of having the depression or related diseases is further configured to utilize a multivariate statistical model to obtain the probability of having the depression or related diseases. Preferably, the multivariate statistical model is a random forest model. According to a preferred embodiment, the probability of having the depression or related diseases greater than a threshold value indicates that the subject suffers from the depression or related diseases or has the risk of developing the depression or related diseases. Preferably, the threshold value is 0.5. According to a preferred embodiment, decrease of the *Bacteroides thetaiotaomicron* and/or the analogue thereof, the *Alistipes shahii* and/or the analogue thereof or the *Prevotella copri* and/or the analogue thereof compared to the reference value indicates that the subject suffers from the depression or related diseases or has the risk of developing the depression or related diseases, and increase of the *Butyriyibrio crossotus* and/or the analogue thereof, the *Clostridium bolteae* and/or the analogue thereof, the *Haemophilus parainfluenzae* and/or the analogue thereof or the *Veillonella dispar* and/or the analogue thereof compared to the reference value indicates that the subject suffers from the depression or related diseases or has the risk of developing the depression or related diseases.

According to a specific embodiment, the device for measuring relative abundance information of a biomarker is further provided with a device for extracting nucleic acids 210, a sequencing device 220 and an alignment device 230, as shown in panel b of Figure 1. According to an embodiment, the device for extracting nucleic acids is configured to extract nucleic acids from the obtained sample of the subject; the sequencing device is connected to the device for extracting nucleic acids and configured to construct a DNA library based on the extracted nucleic acids followed by sequencing to obtain a sequencing result; and the alignment device is connected to the sequencing device and configured to align the sequencing result to a reference gene catalogue to determine the relative abundance information of the biomarker.

According to a specific embodiment, the reference gene catalogue is obtained by subjecting samples from a plurality of patients suffering from the depression or related diseases and a plurality of normal subjects to metagenomic sequencing to obtain a non-redundant gene set, and merging the non-redundant gene set with an intestinal microbial gene set to obtain the reference gene catalogue.

According to embodiments, the sequencing is not particularly limited. Preferably, the sequencing is performed through a second-generation sequencing technology or a third-generation sequencing technology. Preferably, the sequencing is performed by using at least one sequencer selected from Hiseq2000, SOLiD, 454 sequencer and single molecule sequencing sequencers. The sequencer with high-throughput and deep sequencing characteristics used in the present disclosure can be beneficial to the analysis of subsequent sequencing results, especially ensuring the precision and accuracy at statistical testing.

According to an embodiment, the alignment device utilizes at least one of SOAP2 software and MAQ software to perform the alignment, thus improving the efficiency of alignment and increasing the efficiency of depression detection.

In another aspect, the present disclosure in embodiments also proposes a method for screening a medicament. According to an embodiment, the biomarker tightly related to depression is served as a target for screening a medicament for treatment of depression, thus promoting the discovery of new drugs for treating depression. For example, a drug candidate capable of served as a drug for treating or preventing depression can be determined by measuring the change of amount of biomarker(s) before and after contacting with the drug candidate. For another example, whether the amount of harmful biomarker(s) is decreased after contacting with the drug candidate and whether the amount of beneficial biomarker(s) is increased after contacting with the drug candidate are detected. Besides, whether a drug candidate is served as a drug for treating or preventing depression can be determined according to the direct or indirect effect on biological activity of at least one of *Bacteroides thetaiotaomicron* and/or an analogue thereof, *Butyriyibrio crossotus* and/or an analogue thereof, *Alistipes shahii* and/or an analogue thereof, *Clostridium bolteae* and/or an analogue thereof, *Haemophilus parainfluenzae* and/or an analogue thereof, *Veillonella dispar* and/or an analogue thereof and *Prevotella copri* and/or an analogue thereof in presence of the drug candidate. Therefore, the present disclosure in embodiments also proposes use of the biomarker for depression in screening a medicament for treatment or prevention of depression.

It should be noted that the description of terms provided herein is only intended to facilitate skilled in the art to better understand the present disclosure and is not construed as limiting to the present disclosure.

It should be understood that the technical features as described above and the technical features specifically described in the below such as in the examples can be combined with each other to form a new or preferred technical solution within the scope of the present disclosure, which will not be repeated due to the space limitation.

The present disclosure will be described below with reference to specific examples. It should be noted that these examples are merely illustrative and should not be understood as limiting the present disclosure.

The technical means used in the examples are conventional means well known to skilled in the art and can be carried out with reference to the Molecular Cloning: A Laboratory Manual (3rd Edition) or in accordance with the product instructions unless otherwise specified. The reagents or instruments may be commercially available if not specified. The various processes and methods that are not described in detail are conventional methods well known in the art. The source of reagents, the trade name and compositions of which components are necessary to be listed are all indicated when first appearing and the same reagents used thereafter all have the same contents as those first appearing unless otherwise specified.

The present disclosure adopts the metagenome-wide association studies (MWAS) to analyze the microbial species and functional difference of fecal samples and utilizes a random forest model to identify depression population and normal population and obtain the probability of having depression, thus to evaluate the risk of developing depression or related diseases, diagnose or early diagnose depression or related diseases or search for potential drug targets.

According to the present disclosure, the term "Metagenomic Linkage Group (MLG)", refers to a marker artificially set for a same taxonomic unit (such as, strain, species, genus, group or the like) in phylogenetic research or population genetics research so as to facilitate analysis, referring to Qin J, Li Y, Cai Z, et al. A metagenome-wide association study of gut microbiota in type 2 diabetes[J]. Nature, 2012, 490(7418): 55-60. Generally, the sequences are divided into different MLGs according to similarity threshold, and each of MLGs can be regarded as a microbial species. If more than 50% of genes in this MLG can be mapped onto a reference genome with a threshold of 95% identity at the nucleotide level, we considered this particular MLG to originate from this known bacterial species. If more than 50% of genes in this MLG can be mapped onto a reference genome with a threshold of 85% identity at the nucleotide level, we considered this MLG to originate from the same genus of the matched bacterial species.

According to the present disclosure, the term "individual" refers to animal, especially mammal, such as primate, preferably human.

According to the present disclosure, terms such as "a", "an" and "this" refer to not only a singular form, but also a general category used to describe a particular embodiment.

In the present disclosure, the sequencing (such as the second-generation sequencing) and MWAS are well known in the art, which can be adjusted by skilled in the art according to specific conditions. According to embodiments of the present disclosure, the sequencing and MWAS can be performed according to the method described in the literature (Wang, Jun, and Huijue Jia. "Metagenome-wide association studies: fine-mining the microbiome", Nature Reviews Microbiology 14.8 (2016): 508-522).

In the present disclosure, the operation of the random forest model and ROC curve is well known in the art, and the parameters thereof can be set and adjusted by skilled in the art according to specific conditions. According to embodiments of the present disclosure, the random forest model and ROC curve can be operated according to the method described in the literature (Drogan D, Dunn WB, Lin W, Buijsse B, Schulze MB, Langenberg C, Brown M, Floegel a., Dietrich S, Rolandsson O, Wedge DC, Goodacre R, Forouhi NG, Sharp SJ, Spranger J, Wareham NJ, Boeing H: Untargeted Metabolic Profiling Identifies Altered Serum Metabolites of Type 2-Diabetes Mellitus in a Prospective, Nested Case Control Study. Clin Chem 2015, 61:487-497; Mihalik SJ, Michaliszyn SF, de las Heras J, Bacha F, Lee S, Chace DH, DeJesus VR, Vockley J, Arslanian SA: Metabolomic profiling of fatty acid and amino acid metabolism in youth with obesity and type 2 diabetes: evidence for enhanced mitochondrial oxidation. Diabetes Care 2012, 35:605-611), the entire contents of which are incorporated herein by reference.

In the present disclosure, a training set of biomarker(s) for depression patients and normal subjects is constructed, and the amount of biomarker(s) in a sample to be tested can be evaluated based on the training set.

It is known to skilled in the art that when the sample volume is expanded, the amount in normal range (in the absolute value) of each biomarker in the expanded samples can be determined by using testing and calculating methods known in the art. The detected amount in the absolute value of each biomarker in a sample to be tested can be compared to the normal range in the absolute value of each biomarker in the expanded samples, optionally in combination with statistical methods, so as to obtain the efficiency of risk assessment, diagnose depression, monitor the treatment effect on depression patients or the like.

Without wishing to be bound by any theory, the present inventors indicate that these biomarkers are the intestinal microbial species existed in human body. According to association study on intestinal microbes of subjects by the present method, the biomarker for depression shows a certain amount range in depression population via microbe detection.

### Example 1

### 1.1 Sample collection

Fecal samples obtained were transported in a frozen form and stored at -80 °C immediately, followed by DNA extraction to obtain extracted DNA samples, according to the method described in the article "A metagenome-wide association study of intestinal microbiota in type 2 diabetes (Qin, J. et al. Nature 490, 55-60, 2012)". A total of 250 fecal samples of depression subjects and normal subjects were collected from British adult twins, in which 11 samples with missing phenotypes were discarded. The samples with missing phenotypes refer to samples which cannot be diagnosed as depression or cannot be diagnosed by clinical testing. Thus, remaining 239 samples including 160 samples from normal subjects and 79 samples from depression subjects were collected.

### 1.2 Metagenomic sequencing and assembly

A sequencing library was constructed by using the extracted DNAs, followed by paired-end metagenomic sequencing (350bp of insertion size, 100bp of read length) on the Illumina HiSeq2000 sequencing platform. The reads generated by sequencing were filtered, that is quality-controlled by deleting low-quality reads with adapter contamination or human DNA contamination. After that, *de novo* assembly was performed by using the software SOAPdenovo (v2.04) to obtain assembled contigs.

### 1.3 Gene catalogue construction

The assembled contigs were subjected to gene prediction by using the GeneMark (v2.7d). Then the predicted genes were aligned using BLAT and genes, of which over 90% of their length can be aligned to another one with more than 95% identity (no gaps allowed), were removed as redundancies, resulting in a non-redundant gene catalogue comprising of 5,901,478 genes. This non-redundant gene catalogue from the fecal samples was further combined with the previously intestinal microbial reference gene catalogue containing 9,879,896 genes by using software CD-HIT, thereby obtaining an updated gene catalogue containing 11,446,577 genes with 90% or above overlap and 95% or above identity, in reference to the description in the article "A metagenome-wide association study of gut microbiota in type 2 diabetes (Qin, J. et al. Nature 490, 55-60 (2012))".

The high-quality reads for assembly in "1.2 Metagenomic sequencing and assembly" were aligned against the updated intestinal microbial reference gene catalogue containing 11,446,577 genes as described above to obtain the relative abundance of genes, in reference to the method described in the article "A metagenome-wide association study of gut microbiota in type 2 diabetes (Qin, J. et al. Nature 490, 55-60 (2012))".

### 1.4 Taxonomic assignment and annotation and computation of relative abundance

Taxonomic assignment of the predicted genes in 1.3 was performed by aligning with IMG database (v400), in reference to the method described in the article "A metagenome-wide association study of intestinal microbiota in type 2 diabetes (Qin, J. et al. Nature 490, 55-60 (2012))". For taxonomic assignment at the phylum level, the 65% or above identity and 70% or above overlap were used as the threshold for the phylum assignment. For taxonomic assignment at the genus level, the 85% or above identity was used as the threshold for the genus assignment. For taxonomic assignment at the species or strain level, the 95% or above identity was used as the threshold for the species or strain assignment.

The species relative abundance was calculated by using the gene relative abundance, followed by statistical testing with Wilcoxon rank-sum test (p <0.05) to determine the species having significantly different relative abundance between depression subjects and normal subjects, in reference to the method described in the article "A metagenome-wide association study of gut microbiota in type 2 diabetes (Qin, J. et al. Nature 490, 55-60 (2012))".

### 1.5 Biomarker abundance calculation

Co-expressed genes were clustered based on the gene relative abundance; MLGs with more than 50 cluster genes were selected for species annotation; the relative abundance of MLGs was obtained based on the median relative abundance of corresponding genes and MLGs having significantly different relative abundance between depression subjects and normal subjects were determined, in reference to the method described in the article "A metagenome-wide association study of gut microbiota in type 2 diabetes (Qin, J. et al. Nature 490, 55-60 (2012))".

### 1.6 Screening potential biomarker for depression by Random Forest (ROC/AUC)

For further screening the potential biomarker(s) from intestinal microbes, a training set of the biomarker of depression subjects and normal subjects was constructed in this example. The amounts of biomarkers in the samples to be tested were evaluated based on the training set. The training set and the validation set herein have meanings well known in the art. In the example, the training set refers to a dataset including the amounts of individual biomarkers in samples to be tested from a certain number of depression subjects and normal subjects. The validation set refers to an independent dataset used to validate the performance of the training set. Among them, the normal subjects are subjects in good mental state, which may be human or an animal model. The subjects were human in the example, and the experiments were specifically performed by the following steps.

A total of 239 samples including 160 samples from normal subjects and 79 samples from depression subjects were applied in the example. On basis of too less samples from depression subjects, an over-sampling method was performed in reference to the article "Oversampling method for imbalanced classification (Zheng Z, Cai Y, Li Y. Computing and Informatics, 2016, 34(5): 1017-1037)". The 69 samples of depression subjects as an original seed samples were randomly put back and a new 130 samples of depression subjects were selected according to the over-sampling method, and 130 samples were selected from these 160 samples of normal subjects, thereby obtaining 260 samples as the training set. The remaining samples containing 10 samples from depression subjects and 30 samples from normal subjects were served as the validation set.

### 1.6.1 Screening biomarker by using training set data

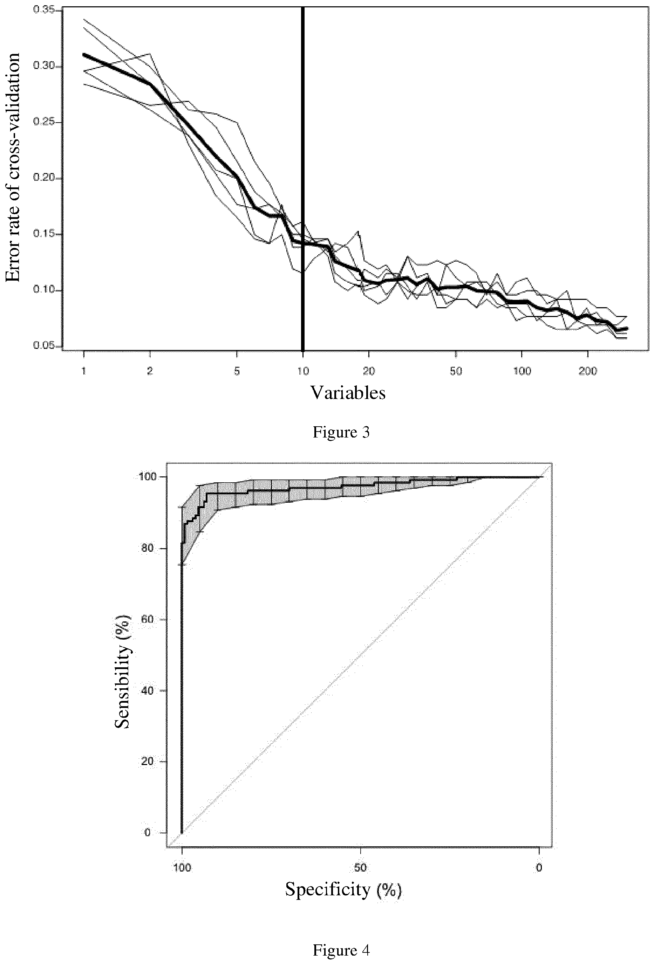

First, the relative abundance of individual genes in each sample of the training set was calculated, followed by gene clustering according to the methods described in 1.4 and 1.5. MLGs with more than 50 genes in the training set were inputted to the random forest classifier (random Forest 4.6-12 in R 3.2.5, RF). 5 times of 10-fold cross verification with 10 times of repetitions were performed on the random forest classifier. The relative abundance of MLGs screened out by using the RF model was used to calculate the risk of developing depression for each sample (refer to Figure 3 and Table 2), and the curve of receiver operation characteristic (ROC) was drawn, thereby obtaining the area under curve (AUC) as the parameter for evaluating discriminant performance of the RF model. The combination of biomarkers which includes less than 30 biomarkers and shows an optimal discriminant performance was selected in the present example. The selection frequency of each MLG was outputted by the RF model. Higher the selection frequency is, more important the MLG as a biomarker is for identifying depression subjects from normal subjects.

The results showed that 8 metabolites (i.e., 8 biomarkers) were obtained according to the RF classier in the present example. The relative abundance of the 8 biomarkers was shown in Table 1, and the detailed information was shown in Table 2. The error rate distribution of the 5 times of 10-fold cross validation in the RF classifier was shown in Figure 3. The RF model was trained by using the relative abundance of qualified MLGs obtained by MWAS process from the samples of the training set (containing 130 samples of depression subjects and 130 samples of normal subjects). In Figure 3, the thick black curve represents the average of 5 tests and the thin black curves independently represent 5 tests. The vertical line represents the number of MLGs in the selected optimal combination. Figure 4 shows the ROC and the AUC of the training set based on the 8 biomarkers by using the RF model to determine depression subjects and normal subjects, in which the specificity represents the probability of identifying normal subjects correctly and the sensibility represents the probability of identifying depression subjects correctly. As shown in Figure 3, the discrimination performance for samples of the train set is: AUC = 97.32% and 95% confidence interval (CI) = 95.37-99.27%. The results indicate that the combination of metabolites obtained by the RF model can be useful as a potential biomarker to identify depression subjects and normal subjects.

In Table 2, the size of gene catalogue of each marker represents the number of nucleic acid sequences for each marker; the annotation number of gene catalogue of markers represents the number of genes annotated to the marker; the optimal annotation of markers represents the corresponding species assignment based on the alignment of all gene catalogues of each marker with the IMG (v400) database; the optimal annotated gene ratio represents the percentage of genes annotated to the target species in the gene cluster; the optimal annotation similarity represents the average of annotation accuracy of all genes in the gene cluster annotated to the target species; the enrichment direction represents the variation of the relative abundance of each biomarker in depression subjects and normal subjects, where D<C represents that the relative abundance of the biomarker in depression subjects is less than that in normal subjects, while C<D represents that the relative abundance of the biomarker in depression subjects is more than that in normal subjects; the screening frequency represents the frequency at which the biomarker is selected after 5 times 10-fold cross validation; the validation set AUC represents the degree of discrimination for the validation set by using the RF model where the RF model is obtained based on the training set data; the 95% confidence interval (95% CI) between a and b represents that, there is a 95% probability that the biomarker is located between a and b and a 5% probability that the biomarker is not located between a and b for each given biomarker.

As can be seen from Table 2, the relative abundance of *Bacteroides thetaiotaomicron* VPI-5482, *Alistipes shahii* WAL 8301 and *Prevotella copri* DSM 18205 in the depression subjects is decreased compared to the normal subjects in the enrichment direction, whereas the relative abundance of *Butyriyibrio crossotus* DSM 2876, *Clostridium bolteae* ATCC BAA-613, *Haemophilus parainfluenzae* ATCC T3T1, *Haemophilus parainfluenzae* ATCC 33392 and *Veillonella dispar* ATCC 17748 in the depression subjects is increased compared to the normal subjects in the enrichment direction.

**Table 1 Relative abundance of intestinal marker MLGs of training set by using RF model**

| Sample ID (D: depression subject; C: normal subject) | Marker number | | | | | | | | | |
|---|---|---|---|---|---|---|---|---|---|---|
| | 24041 | 539 | 716 | 953 | 961 | 1322 | 1335 | 2452 | 5590 | 9956 |
| D_34_1 | 0 | 3.21E-08 | 5.80E-08 | 2.72E-09 | 3.20E-09 | 0 | 0 | 9.85E-08 | 0 | 1.69E-09 |
| D_26_1 | 0 | 0 | 2.84E-09 | 1.66E-09 | 3.46E-09 | 0 | 0 | 9.13E-10 | 0 | 1.05E-06 |
| D_ 52_1 | 1.15E-09 | 6.45E-08 | 4.94E-08 | 5.99E-09 | 6.12E-09 | 0 | 0 | 1.96E-07 | 0 | 0 |
| D_ 39_1 | 0 | 1.93E-09 | 1.05E-08 | 1.30E-08 | 1.68E-08 | 1.25E-08 | 0 | 1.04E-08 | 0 | 0 |
| D_56_1 | 0 | 2.42E-08 | 4.55E-08 | 1.02E-08 | 9.23E-09 | 0 | 0 | 6.93E-08 | 0 | 1.54E-06 |
| D_62_1 | 3.08E-09 | 0 | 2.95E-10 | 3.81E-09 | 6.70E-09 | 1.75E-09 | 2.30E-09 | 1.09E-09 | 0 | 4.32E-07 |
| D_63_1 | 0 | 0 | 0 | 1.21E-08 | 2.18E-08 | 3.94E-08 | 1.19E-10 | 0 | 0 | 1.18E-08 |
| D_ 29_1 | 0 | 1.60E-08 | 2.18E-08 | 7.43E-10 | 3.36E-09 | 1.15E-07 | 0 | 5.08E-08 | 0 | 1.39E-06 |
| D_ 28_1 | 5.86E-10 | 2.72E-08 | 5.42E-08 | 3.22E-08 | 4.07E-08 | 1.53E-08 | 0 | 7.60E-08 | 0 | 8.25E-10 |
| D_36_1 | 1.13E-07 | 0 | 0 | 3.39E-08 | 3.63E-08 | 0 | 0 | 6.20E-11 | 0 | 0 |
| D_66_1 | 0 | 5.40E-08 | 1.02E-07 | 3.74E-08 | 4.03E-08 | 0 | 0 | 1.61E-07 | 0 | 1.73E-07 |
| D_49_1 | 0 | 0 | 0 | 0 | 0 | 0 | 0 | 1.84E-10 | 0 | 0 |
| D_ 32_1 | 0 | 1.73E-09 | 1.79E-09 | 9.42E-08 | 1.12E-07 | 7.15E-09 | 0 | 8.01E-09 | 0 | 0 |
| D_21_1 | 0 | 7.30E-09 | 8.39E-09 | 3.23E-09 | 3.29E-09 | 0 | 0 | 2.51E-08 | 0 | 0 |
| D_38_1 | 0 | 0 | 0 | 0 | 0 | 2.61E-07 | 0 | 0 | 0 | 0 |
| D_67_1 | 0 | 6.70E-09 | 1.32E-08 | 1.77E-08 | 1.72E-08 | 0 | 0 | 2.31E-08 | 0 | 0 |
| D_59_1 | 7.66E-10 | 5.21E-08 | 4.33E-08 | 3.11E-08 | 3.54E-08 | 0 | 1.41E-10 | 1.39E-07 | 0 | 3.07E-07 |
| D_55_1 | 0 | 2.60E-07 | 1.23E-07 | 9.35E-09 | 1.02E-08 | 5.16E-09 | 8.52E-08 | 6.66E-07 | 0 | 0 |
| D_5_1 | 0 | 0 | 0 | 0 | 0 | 0 | 0 | 0 | 0 | 0 |
| D_33_1 | 0 | 3.16E-08 | 2.43E-08 | 4.12E-08 | 4.69E-08 | 0 | 0 | 9.72E-08 | 0 | 1.12E-05 |
| D_31_1 | 3.39E-10 | 0 | 0 | 1.00E-08 | 1.01E-08 | 2.89E-08 | 0 | 0 | 0 | 0 |
| D_37_1 | 0 | 3.86E-08 | 4.84E-08 | 4.70E-09 | 4.84E-09 | 8.87E-09 | 0 | 1.16E-07 | 0 | 0 |
| D_42_1 | 0 | 0 | 0 | 0 | 0 | 2.65E-08 | 0 | 0 | 0 | 3.71E-10 |
| D_16_1 | 0 | 1.76E-07 | 1.77E-07 | 0 | 1.42E-09 | 1.21E-06 | 1.11E-06 | 4.44E-07 | 0 | 0 |
| D_4_1 | 0 | 7.20E-08 | 9.29E-08 | 6.55E-08 | 8.46E-08 | 8.34E-08 | 1.63E-10 | 2.32E-07 | 0 | 4.44E-10 |
| D_58_1 | 1.89E-10 | 1.13E-08 | 1.38E-08 | 1.33E-08 | 1.46E-08 | 0 | 1.73E-09 | 3.42E-08 | 0 | 1.89E-06 |
| D_13_1 | 0 | 0 | 0 | 2.51E-08 | 2.41E-08 | 7.86E-10 | 0 | 2.73E-10 | 0 | 3.31E-09 |
| D_25_1 | 1.28E-09 | 4.42E-10 | 1.58E-09 | 5.06E-08 | 6.82E-08 | 4.76E-08 | 0 | 6.16E-09 | 0 | 0 |
| D_47_1 | 0 | 4.27E-08 | 8.75E-08 | 7.83E-09 | 9.96E-09 | 5.45E-09 | 0 | 1.31E-07 | 0 | 9.85E-06 |
| D_68_1 | 3.58E-06 | 1.60E-08 | 4.57E-08 | 2.99E-07 | 3.58E-07 | 4.50E-09 | 0 | 6.12E-08 | 0 | 0 |
| D_69_1 | 0 | 1.20E-08 | 1.01E-08 | 2.41E-08 | 2.66E-08 | 1.61E-09 | 0 | 4.51E-08 | 0 | 0 |
| D_40_1 | 0 | 1.64E-09 | 4.08E-09 | 9.54E-08 | 1.07E-07 | 1.77E-08 | 0 | 1.15E-08 | 0 | 3.15E-07 |
| D_48_1 | 0 | 1.92E-08 | 1.74E-08 | 0 | 0 | 0 | 0 | 5.60E-08 | 0 | 1.69E-05 |
| D_6_1 | 0 | 1.12E-07 | 1.60E-07 | 3.70E-07 | 3.85E-07 | 0 | 0 | 3.33E-07 | 0 | 3.01E-09 |
| D_65_1 | 5.53E-10 | 1.33E-08 | 8.37E-09 | 2.21E-08 | 2.33E-08 | 0 | 1.29E-06 | 3.72E-08 | 0 | 9.67E-08 |
| D_1_1 | 0 | 2.51E-07 | 2.55E-07 | 2.65E-07 | 2.93E-07 | 4.66E-09 | 2.96E-10 | 6.82E-07 | 0 | 3.29E-10 |
| D_19_1 | 0 | 5.01E-08 | 8.18E-08 | 1.11E-08 | 1.17E-08 | 1.34E-07 | 1.94E-09 | 1.69E-07 | 0 | 0 |
| D_9_1 | 0 | 0 | 0 | 4.00E-09 | 6.54E-09 | 7.14E-08 | 0 | 0 | 0 | 2.08E-10 |
| D_17_1 | 0 | 1.89E-08 | 2.91E-08 | 2.01E-08 | 3.01E-08 | 0 | 1.24E-09 | 5.12E-08 | 0 | 1.37E-10 |
| D_24_1 | 6.21E-10 | 0 | 0 | 1.03E-09 | 2.14E-09 | 2.47E-08 | 4.52E-07 | 2.21E-10 | 0 | 0 |
| D_11_1 | 0 | 9.10E-07 | 5.49E-07 | 1.01E-07 | 1.22E-07 | 8.73E-10 | 0 | 2.11E-06 | 0 | 1.79E-09 |
| D_8_1 | 0 | 6.26E-09 | 8.13E-09 | 6.46E-09 | 1.18E-08 | 0 | 6.61E-10 | 2.40E-08 | 0 | 5.14E-09 |
| D_50_1 | 0 | 1.02E-07 | 1.81E-07 | 2.12E-08 | 2.36E-08 | 3.54E-08 | 0 | 2.95E-07 | 0 | 4.22E-09 |
| D_2_1 | 0 | 8.55E-09 | 1.39E-08 | 7.12E-09 | 1.25E-08 | 6.82E-09 | 0 | 2.87E-08 | 0 | 1.56E-05 |
| D_64_1 | 0 | 0 | 0 | 0 | 0 | 0 | 0 | 0 | 0 | 0 |
| D_45_1 | 0 | 2.95E-07 | 2.68E-07 | 6.36E-08 | 8.76E-08 | 0 | 0 | 7.33E-07 | 0 | 3.85E-06 |
| D_27_1 | 0 | 0 | 0 | 4.42E-09 | 7.64E-09 | 4.64E-09 | 0 | 6.86E-10 | 0 | 2.63E-11 |
| D_7_1 | 5.42E-06 | 6.23E-10 | 4.16E-09 | 9.75E-10 | 1.80E-09 | 0 | 0 | 5.65E-09 | 0 | 2.52E-06 |
| D_60_1 | 0 | 1.35E-07 | 1.21E-07 | 3.64E-08 | 5.57E-08 | 0 | 0 | 3.85E-07 | 0 | 0 |
| D_20_1 | 2.00E-10 | 2.92E-08 | 2.84E-08 | 1.91E-08 | 2.02E-08 | 0 | 0 | 7.16E-08 | 0 | 6.22E-10 |
| D_53_1 | 0 | 1.59E-07 | 1.03E-07 | 5.22E-08 | 8.28E-08 | 7.45E-10 | 0 | 4.41E-07 | 0 | 1.50E-09 |
| D_35_1 | 0 | 3.29E-08 | 3.51E-08 | 6.53E-09 | 6.36E-09 | 0 | 0 | 1.00E-07 | 6.26E-10 | 0 |
| D_23_1 | 0 | 2.28E-08 | 1.60E-08 | 5.98E-08 | 7.80E-08 | 0 | 0 | 6.81E-08 | 0 | 0 |
| D_41_1 | 0 | 8.31E-08 | 8.31E-08 | 1.13E-07 | 1.30E-07 | 5.33E-09 | 0 | 2.26E-07 | 0 | 4.06E-10 |
| D_54_1 | 0 | 5.77E-08 | 5.14E-08 | 1.31E-08 | 1.96E-08 | 0 | 8.76E-08 | 1.70E-07 | 0 | 4.21E-06 |
| D_18_1 | 8.40E-10 | 9.25E-07 | 4.46E-07 | 1.16E-08 | 1.29E-08 | 0 | 3.89E-07 | 2.28E-06 | 3.35E-10 | 3.52E-06 |
| D_46_1 | 0 | 3.60E-07 | 6.13E-07 | 1.08E-06 | 1.39E-06 | 1.49E-09 | 0 | 9.35E-07 | 0 | 2.77E-10 |
| D_30_1 | 0 | 0 | 0 | 0 | 0 | 1.20E-07 | 0 | 0 | 0 | 3.24E-09 |
| D_44_1 | 0 | 3.60E-08 | 9.17E-08 | 0 | 0 | 2.30E-06 | 1.59E-05 | 1.13E-07 | 0 | 4.78E-09 |
| D_51_1 | 1.10E-09 | 7.60E-08 | 7.04E-08 | 5.09E-08 | 6.19E-08 | 1.50E-08 | 0 | 2.12E-07 | 0 | 1.73E-09 |
| D_15_1 | 0 | 8.62E-08 | 1.18E-07 | 8.69E-08 | 1.22E-07 | 1.43E-07 | 0 | 2.37E-07 | 0 | 9.40E-06 |
| D_12_1 | 0 | 1.18E-07 | 1.92E-07 | 1.05E-08 | 1.97E-08 | 0 | 2.19E-10 | 3.62E-07 | 0 | 1.82E-09 |
| D_14_1 | 0 | 3.69E-08 | 4.31E-08 | 0 | 0 | 0 | 0 | 1.14E-07 | 0 | 1.42E-06 |
| D_57_1 | 0 | 2.36E-08 | 1.58E-08 | 1.61E-08 | 1.63E-08 | 0 | 0 | 6.62E-08 | 0 | 4.59E-11 |
| D_3_1 | 0 | 9.47E-10 | 6.80E-10 | 0 | 0 | 0 | 2.38E-10 | 6.01E-09 | 0 | 1.63E-09 |
| D_61_1 | 0 | 0 | 0 | 0 | 2.42E-10 | 2.39E-09 | 0 | 0 | 0 | 0 |
| D_43_1 | 0 | 5.99E-08 | 7.38E-08 | 2.34E-08 | 3.07E-08 | 6.91E-09 | 1.88E-06 | 1.69E-07 | 0 | 1.45E-06 |
| D_10_1 | 4.76E-07 | 3.61E-08 | 2.53E-08 | 3.60E-08 | 3.84E-08 | 0 | 0 | 1.05E-07 | 0 | 0 |
| D_22_1 | 0 | 0 | 0 | 3.00E-09 | 2.98E-09 | 0 | 0 | 0 | 0 | 1.84E-09 |
| D_47_2 | 0 | 4.27E-08 | 8.75E-08 | 7.83E-09 | 9.96E-09 | 5.45E-09 | 0 | 1.31E-07 | 0 | 9.85E-06 |
| D_50_2 | 0 | 1.02E-07 | 1.81E-07 | 2.12E-08 | 2.36E-08 | 3.54E-08 | 0 | 2.95E-07 | 0 | 4.22E-09 |
| D_39_2 | 0 | 1.93E-09 | 1.05E-08 | 1.30E-08 | 1.68E-08 | 1.25E-08 | 0 | 1.04E-08 | 0 | 0 |
| D_41_2 | 0 | 8.31E-08 | 8.31E-08 | 1.13E-07 | 1.30E-07 | 5.33E-09 | 0 | 2.26E-07 | 0 | 4.06E-10 |
| D_32_2 | 0 | 1.73E-09 | 1.79E-09 | 9.42E-08 | 1.12E-07 | 7.15E-09 | 0 | 8.01E-09 | 0 | 0 |
| D_52_2 | 1.15E-09 | 6.45E-08 | 4.94E-08 | 5.99E-09 | 6.12E-09 | 0 | 0 | 1.96E-07 | 0 | 0 |
| D_38_2 | 0 | 0 | 0 | 0 | 0 | 2.61E-07 | 0 | 0 | 0 | 0 |
| D_29_2 | 0 | 1.60E-08 | 2.18E-08 | 7.43E-10 | 3.36E-09 | 1.15E-07 | 0 | 5.08E-08 | 0 | 1.39E-06 |
| D_65_2 | 5.53E-10 | 1.33E-08 | 8.37E-09 | 2.21E-08 | 2.33E-08 | 0 | 1.29E-06 | 3.72E-08 | 0 | 9.67E-08 |
| D_17_2 | 0 | 1.89E-08 | 2.91E-08 | 2.01E-08 | 3.01E-08 | 0 | 1.24E-09 | 5.12E-08 | 0 | 1.37E-10 |
| D_55_2 | 0 | 2.60E-07 | 1.23E-07 | 9.35E-09 | 1.02E-08 | 5.16E-09 | 8.52E-08 | 6.66E-07 | 0 | 0 |
| D_13_2 | 0 | 0 | 0 | 2.51E-08 | 2.41E-08 | 7.86E-10 | 0 | 2.73E-10 | 0 | 3.31E-09 |
| D_53_2 | 0 | 1.59E-07 | 1.03E-07 | 5.22E-08 | 8.28E-08 | 7.45E-10 | 0 | 4.41E-07 | 0 | 1.50E-09 |
| D_6_2 | 0 | 1.12E-07 | 1.60E-07 | 3.70E-07 | 3.85E-07 | 0 | 0 | 3.33E-07 | 0 | 3.01E-09 |
| D_50_3 | 0 | 1.02E-07 | 1.81E-07 | 2.12E-08 | 2.36E-08 | 3.54E-08 | 0 | 2.95E-07 | 0 | 4.22E-09 |
| D_1_2 | 0 | 2.51E-07 | 2.55E-07 | 2.65E-07 | 2.93E-07 | 4.66E-09 | 2.96E-10 | 6.82E-07 | 0 | 3.29E-10 |
| D_21_2 | 0 | 7.30E-09 | 8.39E-09 | 3.23E-09 | 3.29E-09 | 0 | 0 | 2.51E-08 | 0 | 0 |
| D_2_2 | 0 | 8.55E-09 | 1.39E-08 | 7.12E-09 | 1.25E-08 | 6.82E-09 | 0 | 2.87E-08 | 0 | 1.56E-05 |
| D_2_3 | 0 | 8.55E-09 | 1.39E-08 | 7.12E-09 | 1.25E-08 | 6.82E-09 | 0 | 2.87E-08 | 0 | 1.56E-05 |
| D_54_2 | 0 | 5.77E-08 | 5.14E-08 | 1.31E-08 | 1.96E-08 | 0 | 8.76E-08 | 1.70E-07 | 0 | 4.21E-06 |
| D_69_2 | 0 | 1.20E-08 | 1.01E-08 | 2.41E-08 | 2.66E-08 | 1.61E-09 | 0 | 4.51E-08 | 0 | 0 |
| D_2_4 | 0 | 8.55E-09 | 1.39E-08 | 7.12E-09 | 1.25E-08 | 6.82E-09 | 0 | 2.87E-08 | 0 | 1.56E-05 |
| D_2_5 | 0 | 8.55E-09 | 1.39E-08 | 7.12E-09 | 1.25E-08 | 6.82E-09 | 0 | 2.87E-08 | 0 | 1.56E-05 |
| D_34_2 | 0 | 3.21E-08 | 5.80E-08 | 2.72E-09 | 3.20E-09 | 0 | 0 | 9.85E-08 | 0 | 1.69E-09 |
| D_23_2 | 0 | 2.28E-08 | 1.60E-08 | 5.98E-08 | 7.80E-08 | 0 | 0 | 6.81E-08 | 0 | 0 |
| D_40_2 | 0 | 1.64E-09 | 4.08E-09 | 9.54E-08 | 1.07E-07 | 1.77E-08 | 0 | 1.15E-08 | 0 | 3.15E-07 |
| D_29_3 | 0 | 1.60E-08 | 2.18E-08 | 7.43E-10 | 3.36E-09 | 1.15E-07 | 0 | 5.08E-08 | 0 | 1.39E-06 |
| D_9_2 | 0 | 0 | 0 | 4.00E-09 | 6.54E-09 | 7.14E-08 | 0 | 0 | 0 | 2.08E-10 |
| D_15_2 | 0 | 8.62E-08 | 1.18E-07 | 8.69E-08 | 1.22E-07 | 1.43E-07 | 0 | 2.37E-07 | 0 | 9.40E-06 |
| D_7_2 | 5.42E-06 | 6.23E-10 | 4.16E-09 | 9.75E-10 | 1.80E-09 | 0 | 0 | 5.65E-09 | 0 | 2.52E-06 |
| D_17_3 | 0 | 1.89E-08 | 2.91E-08 | 2.01E-08 | 3.01E-08 | 0 | 1.24E-09 | 5.12E-08 | 0 | 1.37E-10 |
| D_7_3 | 5.42E-06 | 6.23E-10 | 4.16E-09 | 9.75E-10 | 1.80E-09 | 0 | 0 | 5.65E-09 | 0 | 2.52E-06 |
| D_17_4 | 0 | 1.89E-08 | 2.91E-08 | 2.01E-08 | 3.01E-08 | 0 | 1.24E-09 | 5.12E-08 | 0 | 1.37E-10 |
| D_12_2 | 0 | 1.18E-07 | 1.92E-07 | 1.05E-08 | 1.97E-08 | 0 | 2.19E-10 | 3.62E-07 | 0 | 1.82E-09 |
| D_14_2 | 0 | 3.69E-08 | 4.31E-08 | 0 | 0 | 0 | 0 | 1.14E-07 | 0 | 1.42E-06 |
| D_65_3 | 5.53E-10 | 1.33E-08 | 8.37E-09 | 2.21E-08 | 2.33E-08 | 0 | 1.29E-06 | 3.72E-08 | 0 | 9.67E-08 |
| D_31_2 | 3.39E-10 | 0 | 0 | 1.00E-08 | 1.01E-08 | 2.89E-08 | 0 | 0 | 0 | 0 |
| D_29_4 | 0 | 1.60E-08 | 2.18E-08 | 7.43E-10 | 3.36E-09 | 1.15E-07 | 0 | 5.08E-08 | 0 | 1.39E-06 |
| D_39_3 | 0 | 1.93E-09 | 1.05E-08 | 1.30E-08 | 1.68E-08 | 1.25E-08 | 0 | 1.04E-08 | 0 | 0 |
| D_57_2 | 0 | 2.36E-08 | 1.58E-08 | 1.61E-08 | 1.63E-08 | 0 | 0 | 6.62E-08 | 0 | 4.59E-11 |
| D_50_4 | 0 | 1.02E-07 | 1.81E-07 | 2.12E-08 | 2.36E-08 | 3.54E-08 | 0 | 2.95E-07 | 0 | 4.22E-09 |
| D_13_3 | 0 | 0 | 0 | 2.51E-08 | 2.41E-08 | 7.86E-10 | 0 | 2.73E-10 | 0 | 3.31E-09 |
| D_42_2 | 0 | 0 | 0 | 0 | 0 | 2.65E-08 | 0 | 0 | 0 | 3.71E-10 |
| D_36_2 | 1.13E-07 | 0 | 0 | 3.39E-08 | 3.63E-08 | 0 | 0 | 6.20E-11 | 0 | 0 |
| D_48_2 | 0 | 1.92E-08 | 1.74E-08 | 0 | 0 | 0 | 0 | 5.60E-08 | 0 | 1.69E-05 |
| D_63_2 | 0 | 0 | 0 | 1.21E-08 | 2.18E-08 | 3.94E-08 | 1.19E-10 | 0 | 0 | 1.18E-08 |
| D_43_2 | 0 | 5.99E-08 | 7.38E-08 | 2.34E-08 | 3.07E-08 | 6.91E-09 | 1.88E-06 | 1.69E-07 | 0 | 1.45E-06 |
| D_43_3 | 0 | 5.99E-08 | 7.38E-08 | 2.34E-08 | 3.07E-08 | 6.91E-09 | 1.88E-06 | 1.69E-07 | 0 | 1.45E-06 |
| D_61_2 | 0 | 0 | 0 | 0 | 2.42E-10 | 2.39E-09 | 0 | 0 | 0 | 0 |
| D_54_3 | 0 | 5.77E-08 | 5.14E-08 | 1.31E-08 | 1.96E-08 | 0 | 8.76E-08 | 1.70E-07 | 0 | 4.21E-06 |
| D_47_3 | 0 | 4.27E-08 | 8.75E-08 | 7.83E-09 | 9.96E-09 | 5.45E-09 | 0 | 1.31E-07 | 0 | 9.85E-06 |
| D_9_3 | 0 | 0 | 0 | 4.00E-09 | 6.54E-09 | 7.14E-08 | 0 | 0 | 0 | 2.08E-10 |
| D_42_3 | 0 | 0 | 0 | 0 | 0 | 2.65E-08 | 0 | 0 | 0 | 3.71E-10 |
| D_27_2 | 0 | 0 | 0 | 4.42E-09 | 7.64E-09 | 4.64E-09 | 0 | 6.86E-10 | 0 | 2.63E-11 |
| D_26_2 | 0 | 0 | 2.84E-09 | 1.66E-09 | 3.46E-09 | 0 | 0 | 9.13E-10 | 0 | 1.05E-06 |
| D_66_2 | 0 | 5.40E-08 | 1.02E-07 | 3.74E-08 | 4.03E-08 | 0 | 0 | 1.61E-07 | 0 | 1.73E-07 |
| D_11_2 | 0 | 9.10E-07 | 5.49E-07 | 1.01E-07 | 1.22E-07 | 8.73E-10 | 0 | 2.11E-06 | 0 | 1.79E-09 |
| D_30_2 | 0 | 0 | 0 | 0 | 0 | 1.20E-07 | 0 | 0 | 0 | 3.24E-09 |
| D_1_3 | 0 | 2.51E-07 | 2.55E-07 | 2.65E-07 | 2.93E-07 | 4.66E-09 | 2.96E-10 | 6.82E-07 | 0 | 3.29E-10 |
| D_7_4 | 5.42E-06 | 6.23E-10 | 4.16E-09 | 9.75E-10 | 1.80E-09 | 0 | 0 | 5.65E-09 | 0 | 2.52E-06 |
| D_35_2 | 0 | 3.29E-08 | 3.51E-08 | 6.53E-09 | 6.36E-09 | 0 | 0 | 1.00E-07 | 6.26E-10 | 0 |
| C_99 | 1.41E-09 | 0 | 0 | 1.40E-07 | 1.76E-07 | 1.30E-08 | 3.49E-09 | 1.23E-11 | 0 | 0 |
| C_13 | 0 | 6.15E-08 | 5.75E-08 | 0 | 8.55E-10 | 0 | 0 | 1.83E-07 | 2.25E-07 | 2.07E-05 |
| C_24 | 4.63E-10 | 0 | 0 | 3.63E-10 | 3.42E-09 | 0 | 0 | 0 | 0 | 3.90E-11 |
| C_16 | 2.58E-09 | 2.01E-08 | 3.04E-08 | 3.05E-08 | 4.22E-08 | 0 | 0 | 6.66E-08 | 0 | 0 |
| C 124 | 3.87E-10 | 4.26E-09 | 7.25E-09 | 0 | 1.13E-10 | 9.47E-10 | 6.91E-07 | 1.53E-08 | 0 | 2.35E-07 |
| C_69 | 8.73E-08 | 0 | 0 | 0 | 0 | 0 | 6.44E-11 | 0 | 0 | 2.00E-11 |
| C_130 | 0 | 5.30E-10 | 2.45E-09 | 1.25E-09 | 2.70E-09 | 5.97E-09 | 0 | 5.81E-09 | 0 | 0 |
| C_114 | 3.91E-10 | 1.04E-08 | 2.63E-08 | 1.28E-09 | 6.00E-09 | 0 | 2.31E-09 | 3.86E-08 | 1.48E-09 | 5.52E-10 |
| C_109 | 0 | 0 | 3.59E-10 | 2.07E-08 | 3.17E-08 | 9.73E-10 | 0 | 6.27E-10 | 0 | 4.14E-07 |
| C_110 | 0 | 4.35E-09 | 4.41E-09 | 1.19E-07 | 1.62E-07 | 0 | 4.55E-09 | 1.62E-08 | 3.46E-09 | 0 |
| C_48 | 5.37E-10 | 0 | 0 | 5.40E-10 | 1.36E-09 | 2.77E-09 | 1.52E-09 | 0 | 0 | 0 |
| C_49 | 0 | 0 | 0 | 1.12E-08 | 2.24E-08 | 0 | 3.03E-11 | 1.37E-09 | 0 | 2.12E-07 |
| C_50 | 4.95E-08 | 0 | 0 | 0 | 0 | 0 | 0 | 0 | 0 | 0 |
| C_25 | 0 | 2.04E-09 | 3.81E-09 | 4.13E-08 | 8.11E-08 | 0 | 2.44E-07 | 8.92E-09 | 0 | 1.80E-06 |
| C_96 | 2.50E-09 | 0 | 0 | 0 | 0 | 0 | 0 | 1.78E-09 | 3.25E-07 | 1.59E-05 |
| C_97 | 0 | 1.05E-09 | 8.80E-10 | 0 | 0 | 0 | 2.61E-11 | 7.73E-09 | 2.20E-06 | 6.28E-07 |
| C_98 | 3.07E-09 | 1.13E-08 | 5.85E-09 | 0 | 6.42E-09 | 4.77E-09 | 5.40E-10 | 3.88E-08 | 0 | 0 |
| C_111 | 7.23E-09 | 2.01E-08 | 1.85E-08 | 4.43E-08 | 6.38E-08 | 0 | 3.71E-07 | 7.29E-08 | 3.58E-10 | 1.02E-06 |
| C_11 | 7.75E-10 | 3.33E-08 | 3.92E-08 | 8.94E-08 | 1.21E-07 | 0 | 0 | 1.15E-07 | 0 | 2.48E-09 |
| C_79 | 0 | 0 | 0 | 0 | 4.52E-11 | 0 | 7.66E-09 | 0 | 0 | 0 |
| C_71 | 0 | 2.06E-06 | 3.55E-06 | 2.07E-06 | 2.58E-06 | 0 | 1.15E-09 | 5.40E-06 | 1.16E-09 | 1.31E-05 |
| C_91 | 2.93E-10 | 0 | 0 | 0 | 4.17E-10 | 3.10E-08 | 7.37E-10 | 0 | 0 | 0 |
| C_10 | 0 | 6.60E-09 | 3.56E-09 | 3.97E-09 | 5.25E-09 | 0 | 9.57E-10 | 2.24E-08 | 0 | 0 |
| C_107 | 6.86E-09 | 6.47E-07 | 8.52E-07 | 3.76E-09 | 8.30E-09 | 0 | 5.61E-10 | 1.68E-06 | 0 | 0 |
| C_121 | 0 | 5.25E-07 | 7.24E-07 | 2.10E-08 | 3.10E-08 | 0 | 0 | 1.19E-06 | 3.70E-08 | 0 |
| C_119 | 5.28E-09 | 2.10E-09 | 2.13E-09 | 4.58E-09 | 7.21E-09 | 0 | 4.06E-09 | 1.21E-08 | 0 | 3.46E-10 |
| C_117 | 3.17E-09 | 0 | 0 | 6.34E-09 | 6.14E-09 | 0 | 6.22E-10 | 1.45E-10 | 0 | 0 |
| C_118 | 4.05E-09 | 0 | 0 | 0 | 4.69E-10 | 7.53E-10 | 2.98E-09 | 0 | 0 | 0 |
| C_41 | 0 | 0 | 0 | 0 | 0 | 0 | 1.78E-10 | 0 | 0 | 0 |
| C_40 | 0 | 6.72E-11 | 0 | 0 | 4.56E-10 | 1.38E-09 | 3.08E-09 | 3.57E-09 | 0 | 0 |
| C_95 | 0 | 2.86E-07 | 5.37E-07 | 7.50E-08 | 7.91E-08 | 1.39E-09 | 1.16E-06 | 7.90E-07 | 1.54E-09 | 1.53E-08 |
| C_93 | 0 | 8.96E-10 | 1.30E-10 | 0 | 0 | 4.52E-09 | 0 | 5.43E-09 | 0 | 2.51E-10 |
| C_92 | 0 | 0 | 0 | 5.28E-10 | 9.87E-10 | 0 | 0 | 0 | 0 | 1.02E-05 |
| C_15 | 2.89E-10 | 0 | 0 | 7.98E-10 | 7.26E-10 | 4.30E-08 | 4.15E-11 | 0 | 0 | 0 |
| C_102 | 0 | 8.27E-08 | 1.01E-07 | 4.05E-08 | 5.32E-08 | 0 | 2.23E-06 | 2.41E-07 | 0 | 0 |
| C_90 | 2.02E-07 | 0 | 3.19E-10 | 0 | 1.88E-09 | 1.24E-08 | 1.10E-10 | 8.78E-10 | 0 | 0 |
| C_43 | 0 | 3.24E-08 | 4.66E-08 | 9.59E-09 | 1.39E-08 | 0 | 4.32E-07 | 1.10E-07 | 0 | 0 |
| C_42 | 0 | 0 | 6.29E-10 | 0 | 0 | 8.87E-09 | 7.69E-10 | 2.39E-10 | 0 | 0 |
| C_108 | 0 | 0 | 0 | 1.90E-09 | 1.42E-09 | 6.03E-08 | 0 | 0 | 0 | 0 |
| C_36 | 0 | 0 | 0 | 1.45E-09 | 4.87E-09 | 8.72E-09 | 2.77E-09 | 8.54E-11 | 0 | 0 |
| C_37 | 4.27E-07 | 0 | 0 | 0 | 0 | 0 | 0 | 1.36E-10 | 0 | 0 |
| C_112 | 6.22E-06 | 4.52E-09 | 1.56E-09 | 2.43E-09 | 1.14E-09 | 4.36E-09 | 1.71E-09 | 1.78E-08 | 0 | 0 |
| C_80 | 0 | 1.42E-07 | 3.54E-07 | 2.26E-06 | 2.53E-06 | 0 | 8.46E-10 | 4.01E-07 | 0 | 1.98E-07 |
| C_106 | 2.52E-08 | 2.04E-09 | 5.45E-09 | 0 | 0 | 0 | 4.03E-10 | 9.70E-09 | 3.03E-09 | 0 |
| C_120 | 1.43E-07 | 3.08E-09 | 3.00E-09 | 0 | 0 | 0 | 3.66E-10 | 1.32E-08 | 0 | 0 |
| C_104 | 0 | 8.38E-10 | 3.85E-09 | 4.07E-09 | 4.29E-09 | 0 | 1.50E-06 | 8.10E-09 | 1.58E-06 | 2.74E-06 |
| C_46 | 3.13E-10 | 0 | 0 | 0 | 0 | 0 | 1.43E-07 | 4.30E-10 | 0 | 1.78E-11 |
| C_123 | 1.74E-09 | 0 | 0 | 0 | 0 | 0 | 0 | 0 | 5.48E-10 | 0 |
| C_54 | 0 | 0 | 0 | 0 | 2.40E-10 | 3.60E-08 | 2.87E-10 | 1.73E-10 | 0 | 0 |
| C_129 | 0 | 2.83E-10 | 1.69E-09 | 0 | 1.45E-09 | 0 | 0 | 3.56E-09 | 0 | 0 |
| C_126 | 2.57E-09 | 1.55E-08 | 3.50E-08 | 7.54E-09 | 8.44E-09 | 0 | 9.55E-11 | 4.38E-08 | 7.57E-08 | 0 |
| C_127 | 1.11E-09 | 0 | 0 | 1.45E-09 | 5.28E-09 | 0 | 5.47E-06 | 2.71E-09 | 0 | 8.55E-10 |
| C_78 | 0 | 1.15E-08 | 1.92E-08 | 0 | 0 | 0 | 0 | 4.28E-08 | 0 | 6.03E-07 |
| C_38 | 0 | 0 | 0 | 0 | 3.83E-10 | 0 | 2.53E-10 | 0 | 0 | 0 |
| C_77 | 2.01E-09 | 0 | 8.79E-10 | 0 | 1.23E-10 | 0 | 0 | 1.47E-09 | 0 | 5.27E-07 |
| C_9 | 5.71E-10 | 2.46E-06 | 2.06E-06 | 4.20E-06 | 5.04E-06 | 0 | 2.06E-06 | 6.19E-06 | 0 | 0 |
| C_12 | 2.55E-09 | 4.21E-08 | 2.12E-08 | 2.83E-09 | 1.05E-08 | 2.50E-09 | 2.40E-09 | 1.57E-07 | 0 | 0 |
| C_70 | 2.38E-08 | 0 | 0 | 0 | 0 | 0 | 0 | 0 | 0 | 0 |
| C_68 | 0 | 1.15E-08 | 8.82E-09 | 1.65E-08 | 1.69E-08 | 5.17E-10 | 2.38E-09 | 4.60E-08 | 0 | 0 |
| C_66 | 0 | 0 | 0 | 0 | 0 | 0 | 3.39E-06 | 0 | 0 | 0 |
| C_67 | 0 | 0 | 0 | 0 | 0 | 0 | 1.38E-08 | 2.76E-10 | 0 | 1.56E-06 |
| C_65 | 0 | 0 | 0 | 0 | 0 | 0 | 0 | 0 | 2.88E-09 | 0 |
| C_72 | 0 | 0 | 0 | 0 | 4.02E-10 | 0 | 1.30E-06 | 0 | 0 | 0 |
| C_19 | 4.58E-09 | 5.81E-07 | 5.21E-07 | 8.82E-08 | 1.19E-07 | 0 | 2.35E-07 | 1.23E-06 | 3.31E-06 | 0 |
| C_51 | 1.76E-08 | 0 | 0 | 0 | 0 | 0 | 0 | 0 | 0 | 2.91E-10 |
| C_14 | 6.20E-10 | 0 | 0 | 0 | 0 | 4.19E-08 | 6.49E-10 | 0 | 0 | 0 |
| C_18 | 0 | 5.15E-08 | 8.29E-08 | 9.94E-09 | 1.55E-08 | 0 | 0 | 1.87E-07 | 0 | 0 |
| C_52 | 0 | 0 | 0 | 0 | 1.77E-09 | 1.04E-08 | 4.57E-09 | 0 | 0 | 2.25E-10 |
| C_53 | 4.64E-07 | 4.26E-09 | 5.82E-09 | 8.46E-08 | 1.16E-07 | 0 | 1.42E-09 | 2.22E-08 | 0 | 0 |
| C_81 | 0 | 5.31E-10 | 2.05E-09 | 1.47E-09 | 1.87E-09 | 2.62E-08 | 5.21E-06 | 4.68E-09 | 0 | 0 |
| C_83 | 1.99E-07 | 0 | 0 | 0 | 8.40E-10 | 4.40E-09 | 2.06E-09 | 0 | 0 | 0 |
| C_82 | 1.34E-06 | 3.74E-08 | 4.42E-08 | 7.99E-09 | 1.00E-08 | 6.71E-10 | 1.80E-10 | 1.22E-07 | 0 | 0 |
| C_125 | 4.18E-07 | 1.52E-09 | 2.16E-09 | 0 | 0 | 0 | 0 | 9.63E-09 | 0 | 0 |
| C_63 | 4.11E-09 | 0 | 1.77E-10 | 0 | 0 | 0 | 4.55E-10 | 7.67E-10 | 0 | 0 |
| C_61 | 5.30E-08 | 8.55E-08 | 3.41E-08 | 3.14E-08 | 3.93E-08 | 0 | 3.16E-11 | 2.57E-07 | 0 | 0 |
| C_62 | 7.33E-10 | 2.45E-07 | 2.70E-07 | 1.09E-08 | 2.64E-08 | 6.07E-10 | 4.35E-09 | 6.15E-07 | 0 | 0 |
| C_59 | 4.23E-09 | 2.24E-09 | 5.72E-09 | 1.38E-09 | 4.24E-09 | 7.15E-09 | 0 | 1.03E-08 | 0 | 0 |
| C_60 | 0 | 0 | 5.33E-10 | 4.01E-09 | 2.91E-09 | 7.13E-09 | 2.01E-09 | 1.37E-09 | 0 | 8.60E-10 |
| C_58 | 0 | 0 | 0 | 0 | 2.65E-09 | 8.43E-09 | 0 | 0 | 0 | 0 |
| C_64 | 0 | 0 | 0 | 0 | 0 | 2.34E-06 | 2.75E-06 | 0 | 0 | 0 |
| C_128 | 3.30E-10 | 0 | 0 | 5.64E-09 | 1.17E-08 | 2.58E-09 | 2.13E-10 | 0 | 0 | 1.38E-08 |
| C_94 | 3.85E-08 | 6.17E-07 | 4.63E-07 | 3.15E-07 | 3.96E-07 | 0 | 0 | 1.49E-06 | 1.09E-09 | 9.06E-09 |
| C_30 | 0 | 0 | 0 | 3.90E-09 | 5.14E-09 | 0 | 7.88E-11 | 0 | 0 | 0 |
| C_31 | 3.08E-09 | 1.62E-08 | 2.58E-08 | 6.39E-08 | 1.05E-07 | 0 | 0 | 5.14E-08 | 0 | 2.83E-09 |
| C_116 | 0 | 7.82E-08 | 1.43E-07 | 8.47E-08 | 1.20E-07 | 0 | 4.04E-10 | 2.51E-07 | 1.00E-09 | 3.86E-09 |
| C_86 | 0 | 0 | 0 | 0 | 0 | 1.57E-08 | 1.22E-10 | 0 | 0 | 0 |
| C_87 | 0 | 0 | 0 | 0 | 0 | 5.01E-09 | 2.20E-06 | 5.75E-11 | 0 | 0 |
| C_84 | 0 | 0 | 0 | 0 | 0 | 1.13E-08 | 1.05E-10 | 0 | 0 | 0 |
| C_85 | 2.86E-10 | 0 | 0 | 0 | 0 | 8.02E-10 | 3.93E-07 | 0 | 0 | 0 |
| C_105 | 0 | 1.46E-07 | 3.62E-07 | 1.07E-07 | 1.27E-07 | 0 | 2.93E-07 | 4.54E-07 | 0 | 0 |
| C_113 | 0 | 0 | 0 | 2.58E-10 | 2.73E-09 | 0 | 0 | 0 | 2.10E-05 | 4.11E-07 |
| C_33 | 0 | 0 | 0 | 1.50E-09 | 1.41E-09 | 9.09E-08 | 0 | 4.57E-10 | 0 | 0 |
| C_32 | 0 | 0 | 0 | 0 | 0 | 1.62E-09 | 6.58E-07 | 0 | 0 | 0 |
| C_73 | 3.38E-09 | 0 | 0 | 0 | 0 | 0 | 0 | 0 | 0 | 0 |
| C_55 | 0 | 3.79E-09 | 3.80E-09 | 0 | 0 | 2.97E-09 | 0 | 1.50E-08 | 0 | 8.54E-10 |
| C_56 | 4.57E-09 | 4.39E-09 | 5.76E-09 | 0 | 0 | 0 | 0 | 1.73E-08 | 0 | 0 |
| C_74 | 0 | 0 | 0 | 1.59E-08 | 2.34E-08 | 0 | 0 | 0 | 0 | 1.95E-06 |
| C_75 | 5.93E-09 | 1.88E-08 | 3.90E-08 | 7.68E-08 | 1.01E-07 | 0 | 0 | 6.48E-08 | 0 | 0 |
| C_57 | 0 | 0 | 0 | 0 | 0 | 4.30E-09 | 6.99E-09 | 0 | 0 | 0 |
| C_34 | 0 | 6.14E-09 | 1.70E-08 | 1.53E-09 | 2.74E-09 | 0 | 1.02E-09 | 3.02E-08 | 0 | 0 |
| C_35 | 5.00E-08 | 0 | 0 | 3.82E-09 | 4.83E-09 | 0 | 0 | 0 | 0 | 0 |
| C_39 | 1.83E-06 | 0 | 0 | 8.01E-09 | 1.07E-08 | 0 | 6.92E-09 | 1.06E-10 | 2.12E-09 | 2.88E-08 |
| C_4 | 2.65E-09 | 1.35E-08 | 1.71E-08 | 0 | 0 | 0 | 0 | 4.49E-08 | 0 | 0 |
| C_29 | 6.00E-09 | 1.62E-09 | 2.82E-09 | 0 | 2.51E-10 | 0 | 0 | 6.31E-09 | 0 | 0 |
| C_28 | 0 | 3.25E-09 | 1.44E-09 | 3.69E-10 | 3.09E-09 | 0 | 1.01E-09 | 1.64E-08 | 0 | 0 |
| C_27 | 1.24E-09 | 1.23E-09 | 1.91E-09 | 1.78E-08 | 1.99E-08 | 0 | 0 | 5.81E-09 | 0 | 8.96E-08 |
| C_26 | 0 | 0 | 0 | 0 | 0 | 0 | 3.07E-10 | 0 | 0 | 1.27E-09 |
| C_122 | 1.08E-09 | 1.63E-07 | 1.11E-07 | 6.09E-08 | 1.13E-07 | 0 | 6.67E-11 | 4.46E-07 | 0 | 0 |
| C_89 | 0 | 0 | 0 | 0 | 0 | 0 | 1.15E-09 | 0 | 4.46E-07 | 0 |
| C_88 | 0 | 2.71E-08 | 2.71E-08 | 1.92E-08 | 2.95E-08 | 0 | 1.67E-07 | 7.52E-08 | 2.15E-06 | 7.71E-07 |
| C_103 | 5.75E-10 | 9.72E-09 | 1.77E-08 | 4.54E-08 | 8.35E-08 | 0 | 3.55E-09 | 3.48E-08 | 2.22E-09 | 3.29E-09 |
| C_20 | 0 | 3.68E-10 | 3.07E-10 | 3.56E-09 | 7.42E-09 | 0 | 0 | 5.54E-09 | 1.85E-06 | 5.08E-09 |
| C_21 | 1.38E-09 | 0 | 0 | 0 | 0 | 1.90E-09 | 8.97E-08 | 0 | 1.40E-07 | 5.09E-06 |
| C_101 | 0 | 7.61E-09 | 1.37E-08 | 4.01E-08 | 5.17E-08 | 0 | 0 | 2.53E-08 | 0 | 0 |
| C_22 | 2.49E-06 | 2.31E-09 | 4.74E-09 | 3.86E-08 | 5.97E-08 | 6.95E-10 | 0 | 1.37E-08 | 0 | 0 |
| C_23 | 2.37E-07 | 1.87E-08 | 4.02E-08 | 1.97E-08 | 3.10E-08 | 0 | 4.55E-07 | 6.07E-08 | 0 | 0 |
| C_100 | 0 | 1.26E-08 | 1.29E-08 | 3.73E-08 | 6.26E-08 | 0 | 7.88E-07 | 4.30E-08 | 1.22E-09 | 4.00E-07 |
| C_115 | 5.10E-09 | 4.95E-11 | 2.18E-09 | 1.33E-08 | 1.74E-08 | 0 | 1.44E-09 | 3.62E-09 | 0 | 0 |
| C_76 | 6.29E-09 | 0 | 0 | 5.36E-09 | 1.05E-08 | 0 | 1.10E-07 | 1.98E-10 | 5.29E-08 | 0 |
| C_47 | 0 | 0 | 0 | 0 | 0 | 0 | 3.62E-10 | 2.05E-10 | 0 | 0 |
| C_8 | 0 | 1.42E-07 | 1.12E-07 | 1.23E-07 | 1.73E-07 | 0 | 0 | 3.76E-07 | 0 | 4.56E-11 |
| C_5 | 0 | 0 | 0 | 0 | 0 | 0 | 0 | 0 | 0 | 0 |
| C_7 | 3.12E-09 | 0 | 0 | 6.25E-08 | 9.32E-08 | 0 | 0 | 0 | 0 | 0 |
| C_6 | 5.49E-09 | 1.49E-07 | 5.75E-08 | 5.87E-09 | 1.61E-08 | 0 | 1.97E-09 | 4.26E-07 | 0 | 0 |
| C_2 | 2.69E-09 | 0 | 0 | 0 | 0 | 0 | 0 | 0 | 0 | 0 |
| C_17 | 0 | 5.91E-09 | 8.19E-09 | 0 | 0 | 0 | 9.99E-07 | 2.79E-08 | 0 | 0 |
| C_1 | 1.53E-10 | 1.12E-09 | 2.28E-09 | 2.36E-09 | 3.41E-09 | 4.73E-08 | 2.40E-09 | 7.94E-09 | 0 | 0 |
| C_45 | 0 | 1.27E-08 | 5.87E-09 | 1.41E-09 | 3.41E-09 | 1.92E-08 | 7.63E-10 | 4.38E-08 | 0 | 0 |
| C_44 | 7.47E-09 | 1.39E-08 | 1.51E-08 | 8.62E-10 | 4.04E-09 | 0 | 4.87E-07 | 4.65E-08 | 5.07E-08 | 2.67E-06 |
| C_3 | 0 | 4.59E-09 | 8.06E-09 | 2.11E-08 | 2.90E-08 | 0 | 2.73E-07 | 2.26E-08 | 0 | 1.10E-09 |

**Table 2 detailed information of 8 biomarkers**

| Mark er numb er | Size of gene catalog ue of markers | Annotati on number of gene catalogue of markers | Optimal annotation of markers (biomarkers information) | Optimal annotated gene ratio | Optimal annotation similarity | Enrichme nt direction (D: depressio n subject; C: normal subject) | Screeni ng frequen cy | Validati on set AUC | 95% CI | Size of gene catalog ue of markers | Sequence source informatio n (sequence number) |
|---|---|---|---|---|---|---|---|---|---|---|---|
| 1335 | 356 | 315 | *Bacteroides thetaiotaomicron* | 0.797752809 | 90.589577 | D<C | 50 | 68.33% | 51.96%-84.7 | 356 | 1∼356 |
| | | | VPI-5482 | | 46 | | | | 1% | | |
| 9956 | 816 | 749 | *Butyrivibrio crossotus* DSM 2876 | 0.915441176 | 99.067322 62 | C<D | 49 | 59.17% | 38.05%-80.2 8% | 816 | 410 ∼ 1225 |
| 24041 | 62 | 48 | *Alistipes shahii* WAL 8301 | 0.709677419 | 93.685227 27 | D<C | 47 | 83.33% | 74.75%-91.9 1% | 62 | 1439 ∼ 1500 |
| 1322 | 66 | 53 | *Clostridium bolteae* ATCC BAA-613 | 0.787878788 | 95.551730 77 | C<D | 47 | 53.67% | 35.3%-72.03 % | 66 | 1501 ∼ 1566 |
| 716 | 71 | 68 | *Haemophilus parainfluenzae* T3T1 | 0.746478873 | 94.521698 11 | C<D | 45 | 55.67% | 34.15%-77.1 8% | 71 | 1644 ∼ 1714 |
| 539 | 655 | 645 | *Haemophilus parainfluenzae* ATCC 33392 | 0.819847328 | 95.411471 14 | C<D | 37 | 60.67% | 43.47%-77.8 6% | 655 | 1715 ∼ 2369 |
| 953 | 72 | 72 | *Veillonella dispar* ATCC 17748 | 0.930555556 | 94.528358 21 | C<D | 37 | 53.83% | 33.48%-74.1 9% | 72 | 2370 ∼ 2441 |
| 2452 | 730 | 726 | *Haemophilus parainfluenzae* ATCC 33392 | 0.882191781 | 96.755729 81 | C<D | 26 | 53.50% | 31.37%-75.6 3% | 730 | 2784 ∼ 3513 |
| 5590 | 51 | 28 | *Prevotella copri* DSM 18205 | 0.549019608 | 95.562857 14 | D<C | 21 | 53.17% | 39.01%-67.3 2% | 51 | 3941 ∼ 3991 |
| 961 | 145 | 145 | *Veillonella dispar* ATCC 17748 | 0.944827586 | 94.414452 55 | C<D | 17 | 64.17% | 45.65%-82.6 8% | 145 | 3992 ∼ 4136 |

Table 3 shows that the combination of 8 biomarkers is used to predict the probability of having depression in the training set, where the probability of 0.5 or above can indicate that the subject is under the risk of having depression or suffers from depression.

**Table 3 Prediction of probability of having depression of training set by using combination of 8 biomarkers**

| Samples ID (D: depression subject; C: normal subject) | Probability of having depression | | Samples ID (D: depression subject; C: normal subject) | Probability of having depression |
|---|---|---|---|---|
| D_1_1 | 0.926315789 | | C_1 | 0.159090909 |
| D_1_2 | 0.92 | | C_2 | 0.459016393 |
| D_1_3 | 0.92513369 | | C_3 | 0.234375 |
| D_2_1 | 0.99382716 | | C_4 | 0.181818182 |
| D_2_2 | 0.994680851 | | C_5 | 0.069518717 |
| D_2_3 | 0.994594595 | | C_6 | 0.118918919 |
| D_2_4 | 0.994413408 | | C_7 | 0.324742268 |
| D_2_5 | 0.994897959 | | C_8 | 0.461111111 |
| D_3_1 | 0.194736842 | | C_9 | 0.360215054 |
| D_4_1 | 0.839378238 | | C_10 | 0.251396648 |
| D_5_1 | 0.698224852 | | C_11 | 0.664835165 |
| D_6_1 | 0.953846154 | | C_12 | 0.358381503 |
| D_6_2 | 0.953608247 | | C_13 | 0.644171779 |
| D_7_1 | 0.942408377 | | C_14 | 0.0625 |
| D_7_2 | 0.93956044 | | C_15 | 0.054644809 |
| D_7_3 | 0.942105263 | | C_16 | 0.424418605 |
| D_7_4 | 0.936416185 | | C_17 | 0.25 |
| D_8_1 | 0.473988439 | | C_18 | 0.783333333 |
| D_9_1 | 0.956989247 | | C_19 | 0.049450549 |
| D_9_2 | 0.956284153 | | C_20 | 0.324742268 |
| D_9_3 | 0.957219251 | | C_21 | 0.107344633 |
| D_10_1 | 0.480662983 | | C_22 | 0.520833333 |
| D_11_1 | 0.895604396 | | C_23 | 0.385869565 |
| D_11_2 | 0.892655367 | | C_24 | 0.13559322 |
| D_12_1 | 0.958115183 | | C_25 | 0.39673913 |
| D_12_2 | 0.96039604 | | C_26 | 0.158823529 |
| D_13_1 | 0.953608247 | | C_27 | 0.345177665 |
| D_13_2 | 0.947976879 | | C_28 | 0.223958333 |
| D_13_3 | 0.949152542 | | C_29 | 0.022346369 |
| D_14_1 | 0.950819672 | | C_30 | 0.123076923 |
| D_14_2 | 0.945121951 | | C_31 | 0.222797927 |
| D_15_1 | 0.981818182 | | C_32 | 0.005434783 |
| D_15_2 | 0.982954545 | | C_33 | 0.404255319 |
| D_16_1 | 0.556756757 | | C_34 | 0.205555556 |
| D_17_1 | 0.951871658 | | C_35 | 0.182741117 |
| D_17_2 | 0.950549451 | | C_36 | 0.058479532 |
| D_17_3 | 0.950276243 | | C_37 | 0.270588235 |
| D_17_4 | 0.951351351 | | C_38 | 0.118918919 |
| D_18_1 | 0.158469945 | | C_39 | 0.116666667 |
| D_19_1 | 0.508379888 | | C_40 | 0.079365079 |
| D_20_1 | 0.966292135 | | C_41 | 0.085427136 |
| D_21_1 | 0.856382979 | | C_42 | 0.104651163 |
| D_21_2 | 0.857142857 | | C_43 | 0.155172414 |
| D_22_1 | 0.383333333 | | C_44 | 0.150289017 |
| D_23_1 | 0.948571429 | | C_45 | 0.351648352 |
| D_23_2 | 0.950276243 | | C_46 | 0.179775281 |
| D_24_1 | 0.105527638 | | C_47 | 0.325842697 |
| D_25_1 | 0.664921466 | | C_48 | 0.232954545 |
| D_26_1 | 0.902173913 | | C_49 | 0.270833333 |
| D_26_2 | 0.902702703 | | C_50 | 0.256544503 |
| D_27_1 | 0.856382979 | | C_51 | 0.231182796 |
| D_27_2 | 0.857894737 | | C_52 | 0.157608696 |
| D_28_1 | 0.817679558 | | C_53 | 0.14893617 |
| D_29_1 | 0.989583333 | | C_54 | 0.315508021 |
| D_29_2 | 0.988095238 | | C_55 | 0.24742268 |
| D_29_3 | 0.990196078 | | C_56 | 0.030150754 |
| D_29_4 | 0.988700565 | | C_57 | 0.038888889 |
| D_30_1 | 0.926315789 | | C_58 | 0.259259259 |
| D_30_2 | 0.926966292 | | C_59 | 0.392473118 |
| D_31_1 | 0.794871795 | | C_60 | 0.418994413 |
| D_31_2 | 0.782608696 | | C_61 | 0.177419355 |
| D_32_1 | 0.849462366 | | C_62 | 0.376963351 |
| D_32_2 | 0.852459016 | | C_63 | 0.127071823 |
| D_33_1 | 0.827777778 | | C_64 | 0.299435028 |
| D_34_1 | 0.984924623 | | C_65 | 0.166666667 |
| D_34_2 | 0.983050847 | | C_66 | 0.020100503 |
| D_35_1 | 0.835164835 | | C_67 | 0.302702703 |
| D_35_2 | 0.825581395 | | C_68 | 0.338797814 |
| D_36_1 | 0.702380952 | | C_69 | 0.165745856 |
| D_36_2 | 0.743455497 | | C_70 | 0.150289017 |
| D_37_1 | 0.592592593 | | C_71 | 0.273255814 |
| D_38_1 | 0.856382979 | | C_72 | 0.184971098 |
| D_38_2 | 0.853932584 | | C_73 | 0.055248619 |
| D_39_1 | 0.971910112 | | C_74 | 0.337423313 |
| D_39_2 | 0.970588235 | | C_75 | 0.283333333 |
| D_39_3 | 0.974619289 | | C_76 | 0.09039548 |
| D_40_1 | 0.765363128 | | C_77 | 0.456647399 |
| D_40_2 | 0.766666667 | | C_78 | 0.50802139 |
| D_41_1 | 0.994252874 | | C_79 | 0.276041667 |
| D_41_2 | 0.994565217 | | C_80 | 0.417647059 |
| D_42_1 | 0.957575758 | | C_81 | 0.154639175 |
| D_42_2 | 0.962962963 | | C_82 | 0.214285714 |
| D_42_3 | 0.961111111 | | C_83 | 0.071428571 |
| D_43_1 | 0.974358974 | | C_84 | 0.087628866 |
| D_43_2 | 0.97382199 | | C_85 | 0.031088083 |
| D_43_3 | 0.970930233 | | C_86 | 0.086021505 |
| D_44_1 | 0.616161616 | | C_87 | 0.097297297 |
| D_45_1 | 0.877777778 | | C_88 | 0.214285714 |
| D_46_1 | 0.803030303 | | C_89 | 0.027027027 |
| D_47_1 | 0.961722488 | | C_90 | 0.087431694 |
| D_47_2 | 0.957219251 | | C_91 | 0.068421053 |
| D_47_3 | 0.95505618 | | C_92 | 0.170454545 |
| D_48_1 | 0.83919598 | | C_93 | 0.327683616 |
| D_48_2 | 0.81920904 | | C_94 | 0.292553191 |
| D_49_1 | 0.305699482 | | C_95 | 0.407960199 |
| D_50_1 | 0.994897959 | | C_96 | 0.258064516 |
| D_50_2 | 0.994444444 | | C_97 | 0.123595506 |
| D_50_3 | 0.994565217 | | C_98 | 0.205405405 |
| D_50_4 | 0.994623656 | | C_99 | 0.346153846 |
| D_51_1 | 0.794117647 | | C_100 | 0.203488372 |
| D_52_1 | 0.923857868 | | C_101 | 0.676136364 |
| D_52_2 | 0.922279793 | | C_102 | 0.235294118 |
| D_53_1 | 0.972067039 | | C_103 | 0.257575758 |
| D_53_2 | 0.971590909 | | C_104 | 0.356382979 |
| D_54_1 | 0.982248521 | | C_105 | 0.404624277 |
| D_54_2 | 0.98265896 | | C_106 | 0.078947368 |
| D_54_3 | 0.982142857 | | C_107 | 0.325966851 |
| D_55_1 | 0.895604396 | | C_108 | 0.194117647 |
| D_55_2 | 0.905940594 | | C_109 | 0.578313253 |
| D_56_1 | 0.947089947 | | C_110 | 0.138554217 |
| D_57_1 | 0.835106383 | | C_111 | 0.4375 |
| D_57_2 | 0.833333333 | | C_112 | 0.128342246 |
| D_58_1 | 0.703296703 | | C_113 | 0.295698925 |
| D_59_1 | 0.845744681 | | C_114 | 0.159763314 |
| D_60_1 | 0.553072626 | | C_115 | 0.186046512 |
| D_61_1 | 0.912568306 | | C_116 | 0.39375 |
| D_61_2 | 0.905325444 | | C_117 | 0.12 |
| D_62_1 | 0.233160622 | | C_118 | 0.039548023 |
| D_63_1 | 0.865 | | C_119 | 0.102564103 |
| D_63_2 | 0.839285714 | | C_120 | 0.070588235 |
| D_64_1 | 0.558974359 | | C_121 | 0.539772727 |
| D_65_1 | 0.923497268 | | C_122 | 0.299465241 |
| D_65_2 | 0.920903955 | | C_123 | 0.047619048 |
| D_65_3 | 0.929292929 | | C_124 | 0.221649485 |
| D_66_1 | 0.97826087 | | C_125 | 0.072727273 |
| D_66_2 | 0.976470588 | | C_126 | 0.204301075 |
| D_67_1 | 0.485 | | C_127 | 0.21875 |
| D_68_1 | 0.50867052 | | C_128 | 0.594594595 |
| D_69_1 | 0.968586387 | | C_129 | 0.386740331 |
| D_69_2 | 0.96969697 | | C_130 | 0.088757396 |

### 1.6.2 Validation of selected biomarkers by using validation set data

In the example, the RF model was validated on independent subjects, where the probability of depression (RP) more than or equal to 0.5 indicates that the subject is under the risk of having depression or suffers from depression. First, the relative abundance of individual biomarker in each sample of the validation set was calculated according to the method described in 1.5, and the validation set data was validated by using the RF model according to the method described in 1.6.1. Table 4 shows the relative abundance of intestinal marker MLGs in the validation set by using the RF model.

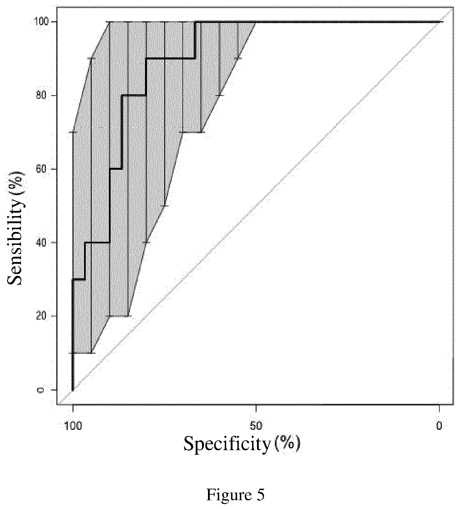

On basis of the RF model, Figure 5 shows the ROC and the AUC of the validation set based on the 8 biomarkers by using the RF model to identify depression subjects and normal subjects, in which the discrimination performance for the independence validation set 1 (10 samples from depression subjects and 30 samples from normal subjects) is AUC=89.67% and 95% confidence interval (CI) = 79.93-99.4% based on the 8 biomarkers. For *Alistipes shahii* WAL 8301, AUC of the validation set is 0.8333, with a high specificity.

Assignment and regression under the RF model were conducted by using "Random Forest 4.6-12 package" in R software (version 3.2.5). The input includes the training set data (i.e., the relative abundance of marker MLGs selected for training samples, refer to Table 1), the disease condition of samples (a vector, in which '1' represents depression and '0' represents normal), and a validation set (i.e., the relative abundance of marker MLGs selected for validation samples, refer to Table 5). After that, the present inventors constructed assignment and prediction functions by using random forest function in "Random Forest 4.6-12 package" of R software to predict the validation set data, and the output is the prediction results (i.e., the probability of having depression), in which the threshold is 0.5 and the probability more than or equal to 0.5 indicates that the subject is under the risk of having depression or suffers from depression.

**Table 4 Relative abundance of intestinal marker MLGs of validation set by using RF model**

| Sample ID (D: depression subject; C: normal subject) | | | Marker number | | | | | | | |
|---|---|---|---|---|---|---|---|---|---|---|
| | 24041 | 1335 | 9956 | 1322 | 539 | 2452 | 716 | 5590 | 961 | 953 |
| C_131 | 1.27E-10 | 0 | 0 | 5.72E-10 | 0 | 0 | 0 | 2.74E-10 | 5.22E-10 | 0 |
| C_132 | 0 | 0 | 4.32E-10 | 2.85E-08 | 2.07E-09 | 8.8E-09 | 9.35E-10 | 8.83E-09 | 0 | 0 |
| C_133 | 1.83E-10 | 0 | 0 | 0.00000073 | 1.25E-08 | 3.62E-08 | 1.54E-08 | 0 | 1.16E-08 | 7.71E-09 |
| C_134 | 9.46E-11 | 0 | 1.28E-09 | 3.48E-08 | 9.17E-10 | 5.38E-09 | 1.41E-09 | 0 | 7.77E-09 | 3.89E-09 |
| C_135 | 1.33E-09 | 3.08E-10 | 6.76E-07 | 0 | 0 | 0 | 0 | 0 | 0 | 0 |
| C_136 | 5.3E-10 | 0 | 0 | 8.51E-10 | 2.48E-09 | 1.18E-08 | 5.36E-09 | 0 | 0 | 0 |
| C_137 | 1.32E-07 | 2.68E-07 | 0 | 4.72E-08 | 0 | 0 | 0 | 0 | 0 | 0 |
| C_138 | 1.37E-10 | 1.04E-09 | 1.62E-11 | 1.91E-09 | 0 | 6.5E-10 | 0 | 0 | 0 | 0 |
| C_139 | 0 | 4.23E-07 | 0 | 0 | 0 | 0 | 0 | 0 | 0 | 0 |
| C_140 | 0 | 2.32E-10 | 0 | 2.6E-09 | 0 | 8.16E-11 | 0 | 0 | 5.19E-11 | 5.2E-10 |
| C_141 | 1.25E-10 | 2.2E-09 | 0.00000632 | 0 | 7.7E-09 | 3.56E-08 | 3.05E-09 | 0 | 1.47E-08 | 7.86E-09 |
| C_142 | 1.94E-10 | 5.88E-09 | 0 | 7.6E-08 | 0 | 0 | 0 | 0 | 0 | 0 |
| C_143 | 0 | 2.99E-09 | 2.33E-09 | 0 | 0 | 1.76E-09 | 0 | 0 | 7.82E-10 | 0 |
| C_144 | 0 | 1.13E-09 | 4.58E-09 | 0 | 0 | 3.76E-10 | 0 | 0 | 7.59E-10 | 2.81E-10 |
| C_145 | 0 | 4.36E-07 | 0 | 0 | 4.28E-07 | 0.00000113 | 4.31E-07 | 0 | 5.35E-08 | 3.84E-08 |
| C_146 | 0 | 7.07E-10 | 0 | 0 | 2.78E-08 | 7.97E-08 | 1.58E-08 | 0 | 1.05E-09 | 0 |
| C_147 | 5.72E-09 | 2.13E-10 | 2.32E-10 | 0 | 0 | 2.56E-10 | 0 | 0 | 6.33E-09 | 4.17E-09 |
| C_148 | 2.15E-09 | 0.00000119 | 3.31E-07 | 0 | 1.41E-07 | 4.14E-07 | 2.74E-07 | 7.42E-09 | 1.76E-07 | 1.32E-07 |
| C_149 | 6.26E-10 | 0 | 0 | 0 | 0 | 0 | 0 | 0 | 1.12E-09 | 6.86E-10 |
| C_150 | 1.17E-09 | 0 | 2.55E-10 | 2.53E-09 | 0 | 0 | 0 | 0 | 7.25E-11 | 0 |
| C_151 | 7.14E-09 | 8.24E-07 | 0 | 0 | 1.12E-08 | 4.91E-08 | 8.26E-09 | 0 | 3.04E-08 | 1.65E-08 |
| C_152 | 5.41E-10 | 0 | 0.0000111 | 0 | 2.19E-08 | 6.32E-08 | 1.18E-08 | 0 | 0 | 0 |
| C_153 | 0 | 0 | 0 | 0 | 7.13E-10 | 6.39E-09 | 1.95E-09 | 0 | 1.89E-07 | 1.46E-07 |
| C_154 | 1.02E-10 | 1.52E-08 | 1.62E-09 | 0 | 3.78E-08 | 1.17E-07 | 2.43E-08 | 3.14E-08 | 1.37E-08 | 5.97E-09 |
| C_155 | 0 | 0 | 2.2E-09 | 2.36E-09 | 0 | 0 | 0 | 0 | 5.39E-09 | 9.72E-09 |
| C_156 | 9.67E-11 | 0 | 4.39E-10 | 0 | 1.42E-09 | 9.07E-09 | 4.47E-09 | 9.53E-09 | 2.23E-08 | 1.49E-08 |
| C_157 | 3.16E-09 | 0 | 0 | 0 | 4.5E-09 | 1.97E-08 | 7.81E-09 | 3.03E-10 | 0 | 0 |
| C_158 | 0 | 4.95E-07 | 2.69E-08 | 0 | 3.26E-08 | 9.54E-08 | 3.89E-08 | 0.00000194 | 6.24E-08 | 4.01E-08 |
| C_159 | 4.52E-10 | 1.38E-09 | 0 | 0 | 9.42E-10 | 9.17E-09 | 9.23E-09 | 0 | 4.23E-08 | 2.01E-08 |
| C_160 | 4.12E-09 | 0 | 0 | 0 | 2.92E-08 | 8.73E-08 | 1.15E-08 | 0 | 6.48E-09 | 7.78E-09 |
| D_70 | 0 | 0.00000339 | 0 | 2.42E-09 | 5.17E-08 | 1.55E-07 | 6.26E-08 | 0 | 8.07E-08 | 6.46E-08 |
| D_71 | 0 | 0 | 3.61E-09 | 2.73E-08 | 3.43E-09 | 1.55E-08 | 5.14E-09 | 0 | 4.43E-08 | 3.08E-08 |
| D_72 | 0 | 0 | 1.18E-08 | 0 | 8.21E-08 | 2.13E-07 | 6.95E-08 | 3.88E-10 | 2.14E-08 | 2.15E-08 |
| D_73 | 0 | 0 | 4.82E-10 | 0 | 1.16E-08 | 3.63E-08 | 1.97E-08 | 0 | 2.49E-09 | 0 |
| D_74 | 0 | 2.56E-10 | 1.78E-08 | 0 | 1.39E-09 | 5.82E-09 | 3.82E-10 | 2.53E-10 | 9.23E-09 | 5.05E-09 |
| D_75 | 0 | 0 | 0.00000237 | 0 | 0 | 0 | 0 | 0 | 0 | 3.32E-10 |
| D_76 | 0 | 0 | 0 | 0 | 0 | 1.09E-10 | 0 | 0 | 0 | 0 |
| D_77 | 0 | 0 | 0 | 3.86E-10 | 0 | 5.08E-10 | 0 | 0 | 6.03E-09 | 8.5E-09 |
| D_78 | 0 | 0 | 0 | 4.45E-08 | 0 | 0 | 0 | 0 | 0 | 0 |
| D_79 | 0 | 0 | 1.89E-09 | 1.95E-09 | 0 | 4.55E-10 | 9.22E-10 | 0 | 7.45E-09 | 3.93E-09 |

**Table 5 Prediction of probability of having the risk of developing depression or suffering from depression in samples from depression subjects and normal subjects by using RF model respectively based on combination of 8 biomarkers and single biomarker Alistipes shahii WAL 8301 (disease probability of 0.5 or above indicates that subject is under the risk of having depression or suffers from depression)**

| Sample ID (D: depression subject; C: normal subject) | Probability of having depression based on combination of 8 biomarkers | Probability of having depression based on biomarker 24041 (*Alistipes shahii WAL* 8301) |
|---|---|---|
| D_79 | 0.434 | 1 |
| D_78 | 0.616 | 1 |
| D_77 | 0.8 | 1 |
| D_76 | 0.456 | 1 |
| D_75 | 0.658 | 1 |
| D_74 | 0.58 | 1 |
| D_73 | 0.358 | 1 |
| D_72 | 0.848 | 1 |
| D_71 | 0.524 | 1 |
| D_70 | 0.442 | 1 |
| C_160 | 0.348 | 0 |
| C_159 | 0.15 | 0.028 |
| C_158 | 0.158 | 1 |
| C_157 | 0.334 | 0.048 |
| C_156 | 0.584 | 0.384 |
| C_155 | 0.47 | 1 |
| C_154 | 0.21 | 0.384 |
| C_153 | 0.642 | 1 |
| C_152 | 0.274 | 0.366 |
| C_151 | 0.052 | 0 |
| C_150 | 0.236 | 0.888 |
| C_149 | 0.344 | 0.75 |
| C_148 | 0.164 | 0 |
| C_147 | 0.41 | 0 |
| C_146 | 0.418 | 1 |
| C_145 | 0.268 | 1 |
| C_144 | 0.118 | 1 |
| C_143 | 0.052 | 1 |
| C_142 | 0.062 | 0.872 |
| C_141 | 0.43 | 0.384 |
| C_140 | 0.02 | 1 |
| C_139 | 0.116 | 1 |
| C_138 | 0.072 | 0.384 |
| C_137 | 0.06 | 0.32 |
| C_136 | 0.228 | 0.366 |
| C_135 | 0.142 | 0.646 |
| C_134 | 0.436 | 0.384 |
| C_133 | 0.586 | 0.872 |
| C_132 | 0.438 | 1 |
| C_131 | 0.382 | 0.384 |

The results as described above show that the present biomarkers exhibit high accuracy and specificity, thus having a good prospect in development of a diagnostic method, and providing a basis for the risk evaluation, disease diagnosis or early diagnosis of depression and searching for potential drug targets of depression.

In the description of the present disclosure, it should be understood that, the terms "center"", "longitudinal", "transverse", "length", "width", "thickness", "upper", "lower", "front", " "back", "left", "right", "vertical", "horizontal", "top", "bottom", "inner", "outer", "clockwise", "counterclockwise", "axial", "radial", "circumferential" or the like indicate the orientation or positional relationship based on the orientation or positional relationship shown in the drawings, which is only for the convenience of describing the present disclosure and simplifying the description, rather than indicating or implying that the device or element should have a specific orientation, or be constructed and operated in a specific orientation, therefore cannot be understood as a limitation of the present disclosure.

In addition, the terms "first" and "second" are only used for descriptive purposes, which cannot be understood as indicating or implying relative importance or implicitly indicating the number of indicated technical features. Therefore, the features defined with "first" and "second" may explicitly or implicitly include at least one of the features. In the description of the present disclosure, "a plurality of" means at least two, such as two, three, or above, unless otherwise specifically defined.

In the description of the present disclosure, unless specified or limited otherwise, the terms "mounted," "connected", "coupled, "fixed" or the like are used broadly, for example, they may encompass a fixed connection, a detachable connection or integrated connection, may be mechanical or electrical mountings or communicate with each other, may be directly or indirectly connected through an intermediate medium, or may further be inner connection of two components. The specific meanings of the terms described above in the present disclosure can be understood according to specific circumstances for ordinary skilled person in the art.

In the present disclosure, unless specified or limited otherwise, a structure in which a first feature is "on" or "below" a second feature may include an embodiment in which the first feature is in direct contact with the second feature, or may also include an embodiment in which the first feature and the second feature are contacted via an additional feature formed there between. Furthermore, a first feature "on," "above," or "on top of" a second feature may include an embodiment in which the first feature is right or obliquely "on," "above," or "on top of" the second feature, or just means that the first feature is at a height higher than that of the second feature; while a first feature "below," "under," or "on bottom of" a second feature may include an embodiment in which the first feature is right or obliquely "below," "under," or "on bottom of" the second feature, or just means that the first feature is at a height lower than that of the second feature.

Reference throughout this specification to "an embodiment", "some embodiments", "an example", "a specific example", or "some examples" means that a particular feature, structure, material, or characteristic described in connection with the embodiment or example is included in at least one embodiment or example of the present disclosure. Thus, the appearances of above phrases in various places throughout this specification are not necessarily referring to the same embodiment or example of the present disclosure. Furthermore, the particular features, structures, materials or characteristics may be combined in any suitable manner in one or more embodiments or examples. In addition, different embodiments or examples described in the specification, as well as features of embodiments or examples, without conflicting, may be combined by one skilled in the art.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A biomarker for depression, comprising at least one selected from the group consisting of
*Bacteroides thetaiotaomicron* and/or an analogue thereof, wherein the genome sequence of the analogue has 85% or above sequence identity with the genome sequence of the *Bacteroides thetaiotaomicron*,
*Butyriyibrio crossotus* and/or an analogue thereof, wherein the genome sequence of the analogue has 85% or above sequence identity with the genome sequence of the *Butyriyibrio crossotus*,
*Alistipes shahii* and/or an analogue thereof, wherein the genome sequence of the analogue has 85% or above sequence identity with the genome sequence of the *Alistipes shahii,*
*Clostridium bolteae* and/or an analogue thereof, wherein the genome sequence of the analogue has 85% or above sequence identity with the genome sequence of the *Clostridium bolteae*,
*Haemophilus parainfluenzae* and/or an analogue thereof, wherein the genome sequence of the analogue has 85% or above sequence identity with the genome sequence of the *Haemophilus parainfluenzae*,
*Veillonella dispar* and/or an analogue thereof, wherein the genome sequence of the analogue has 85% or above sequence identity with the genome sequence of the *Veillonella dispar*, and
*Prevotella copri* and/or an analogue thereof, wherein the genome sequence of the analogue has 85% or above sequence identity with the genome sequence of the *Prevotella copri.*

2. The biomarker according to claim 1, wherein the biomarker is at least one selected from the group consisting of *Bacteroides thetaiotaomicron* VPI-5482, *Butyriyibrio crossotus* DSM 2876, *Alistipes shahii* WAL 8301, *Clostridium bolteae* ATCC BAA-613, *Haemophilus parainfluenzae* ATCC T3T1, *Haemophilus parainfluenzae* ATCC 33392, *Veillonella dispar* ATCC 17748 and *Prevotella copri* DSM 18205.

3. The biomarker according to claim 1 or 2, wherein the genome sequence of the analogue of *Bacteroides thetaiotaomicron* has 95% or above sequence identity with the genome sequence of the *Bacteroides thetaiotaomicron*,
the genome sequence of the analogue of *Butyriyibrio crossotus* has 95% or above sequence identity with the genome sequence of the *Butyriyibrio crossotus,*
the genome sequence of the analogue of *Alistipes shahii* has 95% or above sequence identity with the genome sequence of the *Alistipes shahii,*
the genome sequence of the analogue of *Clostridium bolteae* has 95% or above sequence identity with the genome sequence of the *Clostridium bolteae,*
the genome sequence of the analogue of *Haemophilus parainfluenzae* has 95% or above sequence identity with the genome sequence of the *Haemophilus parainfluenzae*,
the genome sequence of the analogue of *Veillonella dispar* has 95% or above sequence identity with the genome sequence of the *Veillonella dispar*, or
the genome sequence of the analogue of *Prevotella copri* has 95% or above sequence identity with the genome sequence of the *Prevotella copri.*

4. A method of determining a subject suffering from or having a risk of developing depression or related diseases, comprising:
(1) obtaining a sample from the subject,
(2) measuring relative abundance information of the biomarker of any one of claims 1 to 3 in the obtained sample, and
(3) comparing the measured relative abundance information with a reference data set or a reference value.

5. The method according to claim 4, wherein the reference data set comprises relative abundance information of the biomarker of any one of claims 1 to 3 in samples from a plurality of patients suffering from the depression or related diseases and a plurality of normal subjects.

6. The method according to claim 4 or 5, wherein comparing the measured relative abundance information with a reference data set further comprises utilizing a multivariate statistical model to obtain a probability of having the depression or related diseases.

7. The method according to claim 6, wherein the multivariate statistical model is a random forest model.

8. The method according to claim 6 or 7, wherein the probability of having the depression or related diseases greater than a threshold value indicates that the subject suffers from the depression or related diseases or has the risk of developing the depression or related diseases.

9. The method according to claim 8, wherein the threshold value is 0.5.

10. The method according to claim 4, wherein decrease of the *Bacteroides thetaiotaomicron* and/or the analogue thereof, the *Alistipes shahii* and/or the analogue thereof or the *Prevotella copri* and/or the analogue thereof compared to the reference value indicates that the subject suffers from the depression or related diseases or has the risk of developing the depression or related diseases, and
increase of the *Butyriyibrio crossotus* and/or the analogue thereof, the *Clostridium bolteae* and/or the analogue thereof, the *Haemophilus parainfluenzae* and/or the analogue thereof or the *Veillonella dispar* and/or the analogue thereof compared to the reference value indicates that the subject suffers from the depression or related diseases or has the risk of developing the depression or related diseases.

11. The method according to any one of claims 4 to 10, wherein measuring relative abundance information of the biomarker further comprises:
extracting nucleic acids from the obtained sample of the subject,
constructing a DNA library based on the extracted nucleic acids, followed by sequencing to obtain a sequencing result, and
aligning the sequencing result to a reference gene catalogue to determine the relative abundance information of the biomarker.

12. The method according to claim 11, wherein the reference gene catalogue is obtained by:
subjecting samples from a plurality of patients suffering from the depression or related diseases and a plurality of normal subjects to metagenomic sequencing to obtain a non-redundant gene catalogue, and
merging the non-redundant gene catalogue with an intestinal microbial gene catalogue to obtain the reference gene catalogue.

13. The method according to claim 11 or 12, wherein the sample is faeces.

14. The method according to claim 11 or 12, wherein the sequencing is performed through a second-generation sequencing technology or a third-generation sequencing technology.

15. A kit comprising reagents for detecting the biomarker of any one of claims 1 to 3.

16. The kit according to claim 15, further comprising a reference data set or a reference value,
wherein the reference data set or the reference value is served as references for relative abundance information of the biomarker.

17. The kit according to claim 16, further comprising a first computer program product,
wherein the first computer program product is configured to obtain the reference data set or the reference value.

18. The kit according to any one of claims 15 to 17, further comprising a second computer program product,
wherein the second computer program product is configured to perform the method of determining a subject suffering from or having a risk of developing depression or related diseases of any one of claims 4 to 14.

19. Use of the biomarker of any one of claims 1 to 3 in the manufacture of a kit for determining a subject suffering from or having a risk of developing depression or related diseases.

20. The use according to claim 19, wherein determining a subject suffering from or having a risk of developing depression or related diseases further comprises:
(1) obtaining a sample from the subject,
(2) measuring relative abundance information of the biomarker of any one of claims 1 to 3 in the obtained sample, and
(3) comparing the measured relative abundance information with a reference data set or a reference value.

21. The use according to claim 20, wherein the reference data set comprises relative abundance information of the biomarker of any one of claims 1 to 3 in samples from a plurality of patients suffering from the depression or related diseases and a plurality of normal subjects.

22. The use according to claim 20 or 21, wherein comparing the measured relative abundance information with a reference data set further comprises utilizing a multivariate statistical model to obtain a probability of having the depression or related diseases.

23. The use according to claim 22, wherein the multivariate statistical model is a random forest model.

24. The use according to claim 22 or 23, wherein the probability of having the depression or related diseases greater than a threshold value indicates that the subject suffers from the depression or related diseases or has the risk of developing the depression or related diseases.

25. The use according to claim 24, wherein the threshold value is 0.5.

26. The use according to claim 20, wherein decrease of the *Bacteroides thetaiotaomicron* and/or the analogue thereof, the *Alistipes shahii* and/or the analogue thereof or the *Prevotella copri* and/or the analogue thereof compared to the reference value indicates that the subject suffers from the depression or related diseases or has the risk of developing the depression or related diseases, and
increase of the *Butyriyibrio crossotus* and/or the analogue thereof, the *Clostridium bolteae* and/or the analogue thereof, the *Haemophilus parainfluenzae* and/or the analogue thereof or the *Veillonella dispar* and/or the analogue thereof compared to the reference value indicates that the subject suffers from the depression or related diseases or has the risk of developing the depression or related diseases.

27. The use according to any one of claims 20 to 26, wherein measuring relative abundance information of the biomarker of further comprises:
extracting nucleic acids from the obtained sample of the subject,
constructing a DNA library based on the extracted nucleic acids, followed by sequencing to obtain a sequencing result, and
aligning the sequencing result to a reference gene catalogue to determine the relative abundance information of the biomarker.

28. The use according to claim 27, wherein the reference gene catalogue is obtained by:
subjecting samples from a plurality of patients suffering from the depression or related diseases and a plurality of normal subjects to metagenomic sequencing to obtain a non-redundant gene catalogue, and
merging the non-redundant gene catalogue with an intestinal microbial gene catalogue to obtain the reference gene catalogue.

29. Use of a biomarker of any one of claims 1 to 3 as a target for screening a medicament for treatment or prevention of depression or related diseases.

30. Use of a biomarker of any one of claims 1 to 3 in determining a subject suffering from or having a risk of developing depression or related diseases.

31. An apparatus for determining a subject suffering from or having a risk of developing depression or related diseases, comprising
a device for obtaining sample, configured to obtain a sample from the subject,
a device for measuring relative abundance information of a biomarker, connected to the device for obtaining sample and configured to measure relative abundance information of the biomarker of any one of claims 1 to 3 in the obtained sample, and
a device for obtaining a probability of having the depression or related diseases, connected to the device for measuring relative abundance information of a biomarker and configured to compare the measured relative abundance information with a reference data set or a reference value.

32. The apparatus according to claim 31, wherein the reference data set comprises relative abundance information of the biomarker of any one of claims 1 to 3 in samples from a plurality of patients suffering from the depression or related diseases and a plurality of normal subjects.

33. The apparatus according to claim 31 or 32, wherein the device for obtaining a probability of having the depression or related diseases is further configured to utilize a multivariate statistical model to obtain the probability of having the depression or related diseases.

34. The apparatus according to any one of claims 31 to 33, wherein the device for measuring relative abundance information of a biomarker is further provided with
a device for extracting nucleic acids, configured to extract nucleic acids from the obtained sample of the subject,
a sequencing device, connected to the device for extracting nucleic acids, and configured to construct a DNA library based on the extracted nucleic acids followed by sequencing to obtain a sequencing result, and
an alignment device, connected to the sequencing device and configured to align the sequencing result to a reference gene catalogue to determine the relative abundance information of the biomarker.

35. The apparatus according to claim 34, wherein the reference gene catalogue is obtained by:
subjecting samples from a plurality of patients suffering from the depression or related diseases and a plurality of normal subjects to metagenomic sequencing to obtain a non-redundant gene catalogue, and
merging the non-redundant gene catalogue with an intestinal microbial gene catalogue to obtain the reference gene catalogue.

36. A medicament for preventing or treating depression or related diseases, allowing relative abundance of *Bacteroides thetaiotaomicron* and/or the analogue thereof *Alistipes shahii* and/or the analogue thereof or *Prevotella copri* and/or the analogue thereof in a subject to be increased, or
allowing relative abundance of *Butyriyibrio crossotus* and/or the analogue thereof, *Clostridium bolteae* and/or the analogue thereof, *Haemophilus parainfluenzae* and/or the analogue thereof or *Veillonella dispar* and/or the analogue thereof in a subject to be decreased.
